# EUROPÄISCHE PATENTSCHRIFT

(11) **EP 2 021 010 B1**
(45) Veröffentlichungstag und Bekanntmachung des Hinweises auf die Patenterteilung: **23.04.2014**
(21) Anmeldenummer: 07712375.0
(22) Anmeldetag: 28.02.2007
(51) Int. Cl.: A61K 31/775, A61K 31/795, C08G 8/18, C08G 8/24, C08G 8/28, C08G 14/08, C14C 3/20

(54) **KONDENSATIONSPRODUKTE, VERFAHREN ZU DEREN HERSTELLUNG UND DEREN VERWENDUNG IN ARZNEIMITTELN, ALS DESINFEKTIONSMITTEL**
CONDENSATION PRODUCTS, METHOD FOR THEIR PRODUCTION AND USE THEREOF IN MEDICAMENTS, AS DISINFECTANTS
PRODUITS DE CONDENSATION, PROCÉDÉ DE PRODUCTION ASSOCIÉ ET LEUR UTILISATION DANS DES MÉDICAMENTS, EN TANT QUE DÉSINFECTANTS

(30) Priorität: 17.05.2006 EP 06114062; 17.05.2006 EP 06114063
(43) Veröffentlichungstag der Anmeldung: 11.02.2009
(73) Patentinhaber: BASF SE, 67056 Ludwigshafen (DE)
(72) Erfinder: HÜFFER, Stephan, 67063 Ludwigshafen (DE); GARNIER, Sebastien, 69469 Weinheim (DE); REESE, Oliver, 49448 Lemförde (DE); SCHERR, Günter, 67065 Ludwigshafen (DE); ROSER, Joachim, 68161 Mannheim (DE); MROWIETZ, Ulrich, 24119 Kronshagen (DE)
(86) Internationale Anmeldenummer: PCT/EP2007/051887
(87) Internationale Veröffentlichungsnummer: WO 2007/131813

(56) Entgegenhaltungen:
- WO-A-01/21228
- WO-A-91/03226
- WO-A-91/07183
- BE-A- 506 674
- CH-A- 316 965
- CH-A- 339 624
- GB-A- 362 797
- GB-A- 430 343
- US-A- 2 837 563

## Beschreibung

Die Erfindung betrifft ein Kondensationsprodukt (synthetischer Gerbstoff), wie nachfolgend definiert, der gegebenenfalls als Gemisch mit mindestens einem weiteren Gerbstoff vorliegen kann, sowie Verfahren zur Herstellung eines solchen Kondensationsproduktes, dessen Verwendung als Arzneimittel sowie pharmazeutische Zusammensetzungen enthaltend ein solches Kondensationsprodukt. Ein weiterer Gegenstand der vorliegenden Erfindung ist die Verwendung des Kondensationsproduktes als Desinfektionsmittel beispielsweise in Tierstallungen und ein Desinfektionsmittel enthaltend ein solches Kondensationsprodukt.

Gerbstoffe lassen sich prinzipiell in drei Hauptklassen einteilen (siehe Römpps Chemie Lexikon, 9. Auflage (1995), Georg Thieme Verlag Stuttgart, Stichwort "Gerbstoffe", Seiten 1541 bis 1542):
1. anorganische Gerbstoffe wie Chrom(III)-Salze oder Polyphosphate; 2. synthetische organische Gerbstoffe, die meist erhältlich sind durch Sulfonierung löslich gemachter Aldehyd-Kondensationsprodukte von aromatischen Grundkörpern, insbesondere von Phenol, Kresol, Naphthalin und Naphtol; sowie 3. Gerbstoffe von pflanzlichem Ursprung, wie sie in Blättern (Tee), Samen (Kaffee), Beeren, Gallen oder Hölzern vorkommen können. Im engeren Sinne versteht man unter Gerbstoffe von pflanzlichem Ursprung die sogenannten Gerbsäuren beziehungsweise Tannine.

Sowohl die Gerbstoffe pflanzlichen Ursprungs (nachfolgend als pflanzlicher oder natürlicher Gerbstoff bezeichnet) als auch die synthetischen organischen Gerbstoffe (nachfolgend als synthetischer Gerbstoff bezeichnet) werden in der Literatur teilweise mit antiviraler Wirkung in Zusammenhang gebracht. Dies trifft insbesondere auf pflanzliche oder synthetische Gerbstoffe zu, die als sogenannte Polyphenole bezeichnet werden.

Beispielsweise werden für pflanzliche Gerbstoffe wie Tannine in T. Okuda, Phytochemistry, Band 66 (2005), Seiten 2012 bis 2031 oder Fukuji et al., Antiviral Res. 11 (1989), Seiten 285 bis 298 eine antivirale Aktivität (insbesondere gegen Herpes simplex) sowie eine Antitumoraktivität dieser natürlichen Gerbstoffe beschrieben.

Weiterhin wird auch Propolis, das von Bienen aus den Knospen, Rinden und Harzen bestimmter Bäume gesammelt wird und pflanzliche Gerbstoffe enthält, unter anderem eine antivirale Aktivität, beispielsweise gegen Herpes simplex, zugeschrieben. Propolis, das ein komplexes Gemisch ist und u.a. Polyphenol enthält, kann sich je nach Bienenvolk aus bis zu 200 verschiedenen Inhaltsstoffen zusammensetzen, insbesondere sind dies Chalkone, Flavanone, Flavone und Flavanole (S. Bogdanov, Schweizerisches Zentrum für Bienenforschung; Artikel erhältlich aus dem Internet; http://www.apis.admin.ch/de/bienenprodukte/docs/produkte/propolis_d.pdf).

Auch im Fall von synthetischen Gerbstoffen sind pharmazeutische Anwendungen bereits bekannt. So betrifft WO 95/14479 ein Kondensationspolymer aus aromatischen Sulfonsäuren und einem Aldehyd zur Inhibierung des HIV-Virus'. Dort wird beschrieben, dass je höher das Molekulargewicht des Polymers ist, umso größer ist dessen therapeutische Aktivität. Besonders bevorzugt werden durch molekülgrößenabhängige Trennverfahren Kondensationspolymere mit einem M_{w}-Gewicht zwischen 4000 und 12.000 g/mol erhalten. WO 95/14479 offenbart jedoch keine synthetischen Gerbstoffe, die auf Aromaten oder Heteroaromaten mit mindestens einem Carboxy- und mindestens einer Hydroxygruppe beruhen. Sinngemäßes gilt für US-A 4,604,404, worin die Verwendung von beispielsweise sulfonierten Naphthalin-Formaldehyd-Kondensationspolymeren zur Bekämpfung des Herpes simplex-Virus' beschrieben wird. Ähnliche Verbindungen sowie deren Verwendung als Arzneimittel sind in US-A 5,088,400 und US-A 4,364,927 beschrieben.

Weiterhin beschreibt DE-A 33 41 122 äußerlich anzuwendende virucide Arzneimittel, insbesondere gegen Herpes labilis sowie Viruserkrankungen der Haut. Bei diesen Arzneimitteln handelt es sich um synthetische Gerbstoffe, hergestellt durch Kondensation von beispielsweise Harnstoff mit Phenol/Kresol, Formaldehyd sowie einem Sulfonierungsmittel.

In DE-A 10 2004 034613 werden Kondensationsprodukte beschrieben, die erhältlich sind durch Umsetzung von mindestens einem Aromaten, mindestens einem Sulfonierungsmittel, mindestens einer Carbonylverbindung und gegebenenfalls mindestens einem Harnstoffderivat. Im Anschluss an die Synthese werden die Kondensationsprodukte mindestens einem molekülgrößenabhängigen Trennverfahren unterzogen. Dabei wurde das Kondensationsprodukt in drei Fraktionen, eine hochmolekulare, eine mittelmolekulare sowie eine niedermolekulare Fraktion aufgetrennt. Es wurde festgestellt, dass die hochmolekularen Fraktionen eine verbesserte Wirksamkeit hinsichtlich der Inhibierung der Aktivität des Enzyms humane Leukozyten-Elastase aufweisen im Vergleich zu den entsprechenden mittelmolekularen Fraktionen dieser Kondensationsprodukte.

Die deutsche Anmeldung mit der Nummer 10 2005 050 193.1 sowie EP-A 0 301 406 betreffen synthetische Gerbstoffe, insbesondere niedermolekulare Gerbstoffe, für die keine Verwendung als Arzneimittel beschrieben ist.

EP-A 0 301 406 betrifft Kondensationsprodukte, erhältlich durch Umsetzung von Melamin mit Glyoxal oder Glyoxylsäure. Gegebenenfalls wird eine aromatische Verbindung mit einkondensiert, ausgewählt aus Phenolsulfonsäure, Sutfosalicylsäure, Salicylsäure oder 8-Hydroxychinolin. In EP-A 0 301 406 explizit offenbarte Verbindungen sind nicht Gegenstand der vorliegenden Erfindung. Außer in EP-A 0 301 406 wird jedoch in keinem der vorgenannten Dokumente, die synthetische Gerbstoffe (Kondensationsprodukte) als solche auf der Basis von Aromaten oder deren Verwendung als Arzneimittel betreffen, beschrieben, dass die im jeweiligen Kondensationsprodukt eingesetzte Aromatenkomponente Verbindungen sein können, die mit mindestens einer Hydroxy- und mit mindestens einer Carboxygruppe substituiert sind beziehungsweise den Salzen davon.

WO 91/03226 beschreibt pharmazeutische Zusammensetzungen enthaltend aromatische Polymere sowie therapeutische Verfahren zur Verwendung derselben. Dabei wird in Mischungen mit einem pharmazeutisch verträglichen Träger eine therapeutisch effektive Menge einer polyaromatischen polymeren Verbindung eingesetzt, die im Wesentlichen frei von Monomeren ist. Diese Verbindungen zeigen Eigenschaften, welche pharmakologische Aktivitäten von Glycosaminglycanen nachahmen, so dass die Verteilung von biologisch aktiven Peptiden und Proteinen im Gewebe bewirkt werden kann.

WO 91/07183 beschreibt Polymere und diese enthaltende pharmazeutische Zusammensetzungen. Diese biologisch aktiven, polymeren Verbindungen enthalten eine Alkylaryl- oder Heteroalkyl-Aryl Kette mit 5 bis 50 sich wiederholenden aromatischen Einheiten. Sie sind fähig, eine lineare Hauptkette mit einer helikalen Sekundärstruktur zu bilden.

CH 316965 offenbart ein Verfahren zur Herstellung biologisch wirksamer Polykondensationsprodukte aus Gentisinsäure. Dabei wird Gentisinsäure mit Formaldehyd in Anwesenheit einer Säure in einem vorgegebenen Verhältnis kondensiert. Die Kondensationsprodukte haben sich nach Prüfung in vitro und bei Tierversuchen in vivo als Mittel einerseits gegen rheumatoide Poliarthritis und andererseits gegen gewisse klinische Infektionskrankheiten als wirksam erwiesen.

GB 3627979 beschreibt Verbesserungen bei Gerbstoffen, wobei ein Gerbstoff beschrieben ist, der durch Kondensieren eines löslichen Produkts durch die Wirkung von konzentrierter Schwefelsäure auf ein Phenol oder ein Phenolderivat in einer stark sauren Lösung in der Gegenwart von Harnstoff hergestellt wird. Hierdurch wird eine verbesserte Gerbwirkung erreicht.

GB 430,343 offenbart Verbesserungen bei der Herstellung von synthetischen Gerbstoffen. Dabei werden aromatische, sowohl isozyclische als auch heterozyclische Sulfonsäuren mit Aldehyden und aromatischen Hydroxyverbindungen, enthaltend Karbonylgruppen und/oder Halogenatomen kondensiert.

Der Erfindung lag somit die Aufgabe zugrunde, weitere Arzneimittel bereitzustellen, die als antivirales Agens geeignet sind, vorzugsweise sollen diese neuen Arzneimittel eine verbesserte Wirkung gegenüber Viren wie beispielsweise dem Herpes simplex-Virus aufweisen. Erfindungsgemäß wird diese Aufgabe gelöst durch ein Kondensationsprodukt erhältlich durch Umsetzung von
a1) mindestens einem Aromaten oder Heteroaromaten, wobei der Aromat oder Heteroaromat mit mindestens einer Hydroxy- (-OH) und mindestens einer CarboxyGruppe (-COOH) substituiert ist, und wobei die Hydroxy- und/oder CarboxyGruppe in Salzform vorliegen können,
a2) mindestens einer Carbonylverbindung, ausgewählt aus Aldehyden und Ketonen,
a3) mindestens einem Sulfonierungsmittel
a4) mindestens einem Harnstoffderivat, und
a5) mindestens einen weiteren Aromaten oder Heteroaromaten, ausgewählt aus Benzol, Naphthalin, Anthracen, aromatischen Alkoholen, aromatischen Ethern und aromatischen Sulfonen, wobei die vorgenannten Verbindungen substituiert sein können und diese Verbindungen nicht mit mindestens einer Hydroxy- (-OH) und mindestens einer Carboxy-Gruppe (-COOH), oder die Salzform dieser Verbindungen, substituiert sind,
oder ein physiologisch verträgliches Salz davon,
wobei die Umsetzung der einzelnen Komponenten a1) und a2) sowie a3) bis a5) in einer oder in mehreren Stufen erfolgen kann, wobei
zunächst mindestens eine Komponente a1) mit mindestens einer Komponente a3) umgesetzt werden und danach im selben Gefäß ohne vorherige Isolierung mit mindestens einer Komponente a2), mindestens einer Komponente a4) und mindestens einer Komponente a5) umgesetzt werden oder
die Reihenfolge der Zugabe der Komponenten a1) und a5) vertauscht ist oder
die Komponenten a1) und a5) mindestens einmal in Form eines Gemisches zugegeben werden oder
mindestens eine Komponente a1) mit mindestens einer Komponente a3) umgesetzt werden, das Produkt isoliert und dann mit dem Umsetzungsprodukt von mindestens einer Komponente a2), mindestens einer Komponente a4) und mindestens einer Komponente a5) umgesetzt wird,
unter der Voraussetzung, dass die Komponente a1) nicht Salicylsäure oder Sulfosalicylsäure ist, wenn die Komponente a4) Melamin ist,
zur Verwendung als Arzneimittel.

Ein Vorteil der vorliegenden Erfindung ist darin zu sehen, dass die erfindungsgemäßen Kondensationsprodukte eine deutlich verbesserte Wirksamkeit als antivirales Agens, beispielsweise gegenüber dem Herpes simplex-Virus sowie weiteren Viren, aufweisen. Die verbesserte Wirksamkeit wird insbesondere dadurch erzielt, dass ein Kondensationsprodukt hergestellt wird aus mindestens einem Aromaten oder Heteroaromaten, der mit mindestens einer Hydroxy- und mindestens einer Carboxygruppe substituiert ist (Komponente a1)), wobei die Hydroxy- und/oder Carboxygruppe auch teilweise oder vollständig in Salzform vorliegen können. Insbesondere tritt eine deutlich verbesserte Wirksamkeit bei der Verwendung von Gallussäure oder Salzen der Gallussäure auf. Die verbesserte Wirksamkeit der erfindungsgemäßen Kondensationsprodukte liegt sowohl gegenüber herkömmlichen synthetischen Gerbstoffen auf Phenolbasis als auch gegenüber pflanzlichen Gerbstoffen auf Gallussäurebasis, die beispielsweise in grünem Tee enthalten sind, vor.

Ein weiterer Vorteil der erfindungsgemäßen Gemische ist darin zu sehen, dass in dem Fall, in dem ein erfindungsgemäßes Kondensationsprodukt auf Formaldehyd-Basis als Gemisch mit einem weiteren synthetischen, formaldehydfreien Gerbstoff eingesetzt wird, insbesondere unter Verwendung von mindestens einem Kondensationsprodukt (C) oder (D), der Anteil an formaldehydhaltigen Komponenten in dem erfindungsgemäßen Gemisch verringert werden kann. Die Aktivität dieser Gemische ist in Abhängigkeit vom Gehalt an erfindungsgemäßem Kondensationsprodukt zumindest gleich, in der Regel jedoch besser als bei synthetischen Gerbstoffen gemäß dem Stand der Technik. Dies ist vor dem Hintergrund zu sehen, dass Formaldehyd, das ein weit verbreitetes Edukt bei der Herstellung von synthetischen Gerbstoffen ist, von der Weltgesundheitsbehörde (WHO) mittlerweile als kanzerogenverdächtig eingestuft wird. Demzufolge sollte Formaldehyd bei der Synthese möglichst vermieden werden, da bei den erhaltenen Kondensationsprodukten immer ein gewisser Formaldehyd-Restgehalt freigesetzt wird. Da aber beispielsweise auch ein Apfel in geringen Konzentrationen Formaldehyd enthält, sind dementsprechend geringe Formaldehyd-Konzentrationen in pharmazeutischen Zusammensetzungen tolerabel. Durch diese Ausführungsformen der erfindungsgemäßen Kondensationsprodukte wird jedoch der Formaldehydgehalt reduziert.

Nachfolgend wird das erfindungsgemäße Kondensationsprodukt näher definiert.

Das Kondensationsprodukt ist erhältlich durch Umsetzung der nachfolgenden Komponenten a1) und a2) sowie a3) bis a5).
*a1) mindestens ein Aromat oder Heteroaromat, wobei der Aromat oder Heteroaromat mit mindestens einer Hydroxy- (-OH) und mindestens einer Carboxygruppe (-COOH) substituiert ist, und wobei die Hydroxy- und*/*oder Carboxygruppe in Salzform vorliegen können*

Nachfolgend werden zunächst die Begriffe Aromat und Heteroaromat definiert ohne Berücksichtigung der in jedem Aromat oder Heteroaromat jeweils zwingend vorhandenen Substituenten (Gruppen) Hydroxy (-OH) und Carboxy (-COOH) oder die entsprechende Salzform dieser beiden Substituenten.

Unter Aromat sind Verbindungen mit mindestens einem Phenylring zu verstehen, der substituiert sein kann und der auch mehrere kondensierte Phenylsysteme umfassen kann, beispielsweise Naphthylsystem, Phenanthrensysteme und Anthracensysteme. gegebenenfalls können bei bi- oder polycyclischen Systemen auch einzelne Cyclen ganz oder teilweise gesättigt sein, vorausgesetzt, dass mindestens ein Zyklus aromatisch ist. Bevorzugte Beispiele für Aromaten sind Benzol, Naphthalin und Anthracen.

Als Heteroaromat werden in der vorliegenden Erfindung aromatische Systeme bezeichnet, die vorzugsweise monocyclische oder bicyclische, gegebenenfalls auch polycyclisch sind und die mindestens ein Heteroatom enthalten, vorzugsweise ausgewählt aus Stickstoff, Sauerstoff und Schwefel. Beispiele für einen Heteroaromaten sind: Pyrrol, Furan, Thiophen, Imidazol, Pyrazol, 1,2,3-Triazol, 1,2;4-triazol, 1,3-Oxazol (= Oxazol), 1,2-Oxazol (= Isoxazol), Oxadiazol, 1,3-Thiazol (= Thiazol), 1,2-Thiazol (= Isothiazol), Tetrazol, Pyridin, Pyridazin, Pyrimidin, Pyrazin, 1,2,3-Triazin, 1,2,4-Triazin, 1,3,5-Triazin, 1,2,4,5-Tetrazin, Indazol, Indol, Benzothiophen, Benzofuran, Benzothiazol, Benzimidazol, Chinolin, Isochinolin, Chinazolin, Cinnolin, Chinoxalin, Phthalazin, Thienothiophen, 1,8-Naphthyridin, andere Naphthyridine, Purin oder Pteridin.

Sofern es sich nicht um monocyclische Systeme handelt, ist bei jedem der vorgenannten Heteroaromaten für den zweiten Ring auch die gesättigte Form (Perhydroform) oder die teilweise ungesättigte Form (beispielsweise die Dihydroform oder Tetrahydroform) oder die maximal ungesättigte (nicht-aromatische) Form mit umfasst, sofern die jeweiligen Formen bekannt und stabil sind. Die Bezeichnung Heteroaromat umfasst somit in der vorliegenden Erfindung beispielsweise auch Bi- oder Polycyclen, in denen (im Falle des Bicyclus) sowohl beide Ringe aromatisch sind als auch Bicyclen, in denen nur ein Ring aromatisch ist.

Solche Beispiele für Heteroaromaten sind: 3H-Indolin, 2(1H)-Chinolinon, 4-Oxo-1,4-dihydrochinolin, 2H-1-Oxoisochinolin, 1,2-Dihydrochinolin, 3,4-Dihydrochinolin, 1,2-Dihydroisochinolinyl, 3,4-Dihydroisochinolin, Oxindolyl, 1,2,3,4-Tetrahydroisochinolin, 1,2,3,4-Tetrahydrochinolin, 5,6-Dihydrochinolin, 5,6-Dihydroisochinolin, 5,6,7,8-Tetrahydrochinolin oder 5,6,7,8-Tetrahydroisochinolin.

Außer den zwingend vorhandenen Substituenten (mindestens einmal) Hydroxy und (mindestens einmal) Carboxy können die in Komponente a1) enthaltenen Aromaten oder Heteroaromaten gegebenenfalls mindestens einen weiteren Substituenten aufweisen. Sofern ein oder mehrere weitere Substituenten vorhanden sind, werden diese unabhängig voneinander gewählt aus:
C₁-C₁₀-Alkylgruppen wie beispielsweise wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; besonders bevorzugt C₁-C₄-Alkyl wie Methyl, Ethyl, n-Propyl, iso-Propyl, n-Butyl, iso-Butyl, sec.-Butyl und tert.-Butyl,
C₂-C₁₀-Alkenylgruppen, insbesondere Vinyl, 1-Allyl, 3-Allyl, 2-Allyl, cis- oder trans-2-Butenyl, ω-Butenyl,
C₆-C,₄-Arylgruppen, wie beispielsweise Phenyl, 1-Naphthyl, 2-Naphthyl, 1-Anthryl, 2-Anthryl,-9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl,
oder Benzylgruppen.

Vorzugsweise enthält die Komponente a1) einen Aromat, bevorzugt Benzol. Komponente a1) ist somit insbesondere mindestens ein Benzol, das mit mindestens einer Hydroxy- und mindestens einer Carboxygruppe substituiert ist, wobei die Hydroxy- und/oder Carboxygruppe in Salzform vorliegen können.

Bevorzugt ist die Komponente a1) ausgewählt aus Gallussäure (Trihydroxybenzoesäure), Salicylsäure oder Gemische davon, wobei die einzelnen Komponenten auch teilweise oder vollständig in Form von Salzen eingesetzt werden können, vorzugsweise als physiologisch verträgliche Salze. Bevorzugte physiologisch verträgliche Salze von Komponente a1) sind Alkalimetallsalze (insbesondere das Natriumsalz "Natriumgallat" oder das Kaliumsalz), und Erdalkalisalze (z.B. Magnesiumsalz). Weitere bevorzugte Salze sind Bismutsalze, zum Beispiel Bismut(III)Gallat. Besonders bevorzugt ist die Komponente a1) Gallussäure. Gegebenenfalls kann Gallussäure in Form ihrer Salze (Gallate) eingesetzt werden.

### a2) mindestens eine Carbonylverbindung

Die Carbonylverbindung ist ausgewählt aus Aldehyden und Ketonen, bevorzugt mit mindestens einem Aldehyd wie Formaldehyd, Acetaldehyd oder Propionaldehyd und insbesondere mit Formaldehyd. Wünscht man Formaldehyd einzusetzen, so ist es bevorzugt, Formaldehyd in wässriger Lösung einzusetzen.

### a3) mindestens einem Sulfonierungsmittel

Geeignete Sulfonierungsmittel sind beispielsweise Schwefelsäure, insbesondere konzentrierte Schwefelsäure, weiterhin Oleum mit einem Gehalt an SO₃ von 1 bis 30 Gew.%, weiterhin Chlorsulfonsäure und Amidosulfonsäure. Bevorzugt sind konzentrierte Schwefelsäure und Oleum mit einem Gehalt an SO₃ von 1 bis 15 Gew.-°%.

### a4) mindestens einem Harnstoffderivat

Prinzipiell sind als Komponente a4) Harnstoff und alle Derivate davon geeignet. Bevorzugt ist ein Hamstoffderivat, das an jedem Stickstoffatom mindestens ein Wasserstoffatom trägt.

Besonders bevorzugt wird mindestens einem Harnstoffderivat gewählt aus Verbindungen der allgemeinen Formel (I) 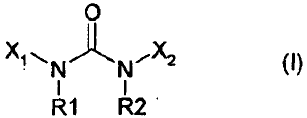 in denen die Variablen wie folgt definiert sind:
X¹, X² sind verschieden oder vorzugsweise gleich und gewählt aus Wasserstoff und -CH₂OH,
R¹, R² sind verschieden oder vorzugsweise gleich und gewählt aus Wasserstoff, C₁-C₁₀-Alkyl wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethytpropyl, iso-Amyl, n-Hexyl, iso-Hexyl, sec.-Hexyl, n-Heptyl, n-Octyl, 2-Ethylhexyl, n-Nonyl, n-Decyl; besonders bevorzugt C₁-C₄-Alkyl wie Methyl, Ethyl, n-Propyl, iso-Propyl, n-Butyl, iso-Butyl, sec.-Butyl und tert.-Butyl, oder
R¹ und R² bilden zusammen eine C₂-C₁₀-Alkyleneinheit, unsubstituiert oder substituiert mit 2 bis 5 Hydroxylgruppen, wie beispielsweise -(CH₂)₂-, -CH₂-CH(CH₃)-, -(CH₂)₃-, -CH₂-CH(C₂H₅)-, -(CH₂)₄-, -(CH₂)₅-, (CH₂)₈-, -(CH₂)₇-, -(CH₂)₈-, -(CH₂)₉-, -(CH-OH)₂-(cis oder trans), vorzugsweise C₂-C₄-Alkylen; insbesondere -(CH₂)₂-, -(CH₂)₃-, und -(CH-OH)₂- (cis oder trans).

Ganz besonders bevorzugt sind (unsubstituierter) Harnstoff, Melamin oder die cyclischen Harnstoffderivate der Formeln 1.1, 1.2 oder 1.3

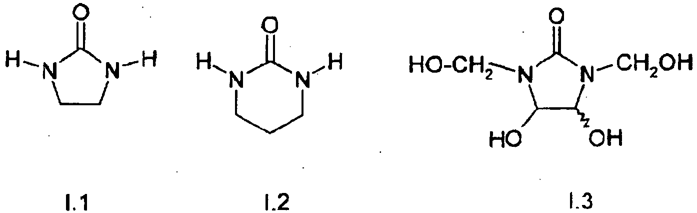

### a5) mindestens einen weiteren Aromaten oder Heteroaromaten

Die Komponente a5) umfasst alle Aromaten und Heteroaromaten, die nicht unter die Definition von Komponente a1) fallen. Komponente a5) umfasst somit alle Aromaten und Heteroaromaten, die nicht mit mindestens einer Hydroxy-(-OH) und mindestens einer Carboxygruppe (-COOH) substituiert sind (oder die Salzformen dieser Verbindungen). Die Definition der Begriffe "Aromat" und "Heteroaromat" entspricht denjenigen von Komponente a1). Komponente a5) umfasst demzufolge auch unsubstituierte Aromaten wie Benzol, mindestens eine Hydroxygruppe, aber keine Carboxygruppe aufweisende Verbindungen wie Phenol oder Resorcin sowie mindestens eine Carboxygruppe, aber keine Hydroxygruppe aufweisende Verbindungen wie Benzoesäure oder Phthalsäure.

Bevorzugt ist Komponente a5) mindestens ein Aromat oder Heteroaromat ausgewählt aus Benzol, Naphthalin, Anthracen, aromatischen Alkoholen, aromatischen Ethern und aromatischen Sulfonen, wobei die vorgenannten Verbindungen gegebenenfalls substituiert sein können.

Beispiele für bevorzugte Aromaten oder Heteroaromaten gemäß Komponente a5) sind:

Benzol, Toluol, ortho-Xylol, meta-Xylol, para-Xylol, Ethylbenzol, Cumol, para-Methylcumol, Biphenyl, 2-Methylbiphenyl, 3-Methylbiphenyl, 4-Methylbiphenyl, Bitolyl (4,4'-Dimethylbiphenyl), para-Terphenyl, Inden, Fluoren, Methylindene (Isomerengemisch). Naphthalin, 1-Methylnaphthalin, 2-Methylnaphthalin, 1,8-Dimethylnaphthalin, 2,7-Dimethylnaphthalin, Phenanthren, Anthracen, 9-Methylanthracen, 9-Phenylanthracen.

Als Beispiele für aromatische Alkohole seien genannt: Phenol, ortho-Kresol, meta-Kresol, para-Kresol, 2-Ethylphenol, 3-Ethylphenol, 4-Ethylphenol, 2,3-Dimethylphenol, 2.4-Dimethylphenol, 2,5-Dimethylphenol, 2,6-Dimethylphenol, 3,4-Dimethylphenol, 3,5-Dimethylphenol, α-Naphthol, β-Naphthol, 9-Hydroxyanthracen als Tautomeres von Anthron, 9-Hydroxyphenanthren, Diphenylmethan, Phenyt-(2-methylphenyl)methan, Phenylparatolylmethan, Phenylmetatolylmethan.

Als Beispiele für aromatische Ether seien genannt: Diphenylether, Di-ortho-Tolylether, Di-meta-Tolylether und Di-para-Tolyiether.

Als Beispiele für aromatische Sulfone seien Diphenylsulfon und Dihydroxydiphenylsulfon, insbesondere 4,4'-Dihydroxydiphenylsulfon genannt.

Besonders bevorzugt ist die Komponente a5) Phenol.

In einer Ausführungsform der vorliegenden Erfindung setzt man als Komponente a5) Mischungen aus mindestens 2 Aromaten ein, beispielsweise Mischungen von Naphthalin und Phenol, Naphthalin und Kresol (Isomerengemisch), Naphthalin und Diphenylether, Naphthalin und Ditolylether oder Phenol und Ditolylether.

In einer bevorzugten Ausführungsform ist das erfindungsgemäße Kondensationsprodukt erhältlich durch Umsetzung von
a1) Gallussäure oder einem Salz der Gallussäure,
a2) mindestens einem Aldehyd ausgewählt aus Formaldehyd, Acetaldehyd und Propionaldehyd
a3) konzentrierter Schwefelsäure
a4) mindestens einem Hamstoffderivat ausgewählt aus Harnstoff, Melamin, 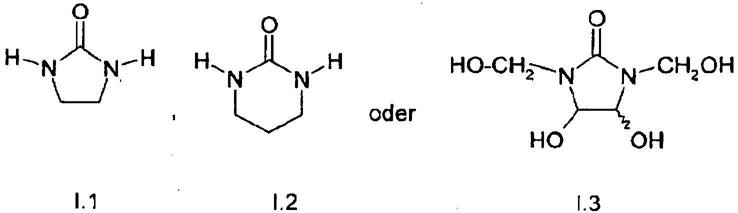
a5) Phenol

Verfahren zur Herstellung eines erfindungsgemäßen Kondensationsproduktes sind dem Fachmann bekannt, beispielsweise werden sie in EP-A 37 250, DE-A 1 113 457, Ullmanns Encyclopedia of Industrial Chemistry, Band A15, (5.Auflage) Weinheim 1990, S.259 - 282 oder DE-A 848 823 beschrieben.

Ullmanns Encyclopedia of Industrial Chemistry, Band A15, (5.Auflage) Weinheim 1990, S.259 - 282 oder DE-A 848 823 beschrieben.

Man kann dabei die einzelnen Komponenten a1) und a2) sowie a3) bis a5) in einer oder in mehreren Stufen umsetzen. Beispielsweise kann man zunächst
a1) mindestens einen mit -COOH und -OH substituierten Aromaten oder Heteroaromaten
a3) mit mindestens einem Sulfonierungsmittel umsetzen und danach im selben Gefäß ohne vorherige Isolierung mit
a2) mindestens einer Carbonylverbindung,
a4) mindestens einem Hamstoffderivat und
a5) mindestens einem weiteren Aromaten oder Heteroaromaten umsetzen.

Alternativ kann dabei die Reihenfolge der Zugabe der Komponenten a1) und a5) vertauscht sein oder a1) und a5) werden mindestens einmal in Form eines Gemisches zugegeben.

Man kann in einer anderen Ausführungsform vorgehen, indem man
a1) mindestens einen mit -COOH und -OH substituierten Aromaten oder Heteroaromaten
a3) mit mindestens einem Sulfonierungsmittel umsetzt, das Produkt isoliert und dann mit dem Umsetzungsprodukt von
a2) mindestens einer Carbonylverbindung,
a4) mindestens einem Harnstoffderivat und
a5) mindestens einem weiteren Aromaten oder Heteroaromaten umsetzt.

Man kann in einer Ausführungsform der vorliegende Erfindung die Komponenten a1) und a2) und gegebenenfalls a3), a4) und a5) in jeweils einer Portion zur Umsetzung bringen.

In einer anderen Ausführungsform der vorliegende Erfindung bringt man mindestens eine Komponente a1) bis a5) in mindestens zwei Portionen zur Umsetzung. Vorzugsweise sind dies die Komponenten a1), a2) und/oder a5), insbesondere die Komponente a2). Dies bedeutet, dass man die zweite Portion - in maximal äquimolaren Mengen zur ersten Portion - nach Umsetzung der übrigen Komponenten in das Reaktionsgefäß zugibt und eine sogenannte Nachkondensation durchführt.

In einer Ausführungsform der vorliegenden Erfindung kann man während der Umsetzung
a6) einen oder mehrere weitere Komponenten zusetzen, beispielsweise NaHSO₃, Na₂S₂O₅, KHSO₃, K₂S₂O_{5,} wässrige Alkalimetallhydroxidlösung, insbesondere wässrige Natronlauge und wässrige Kalilauge, und wässeriges Ammoniak. Weiterhin sind auch Komplexbildner als Komponente a6) geeignet, beispielsweise Komplexbildner auf Basis Ethylendiamin-Tetraessigsäure. Ein Beispiel für einen solchen Komplexbildner ist das Handelsprodukt Trilon B (BASF AG, Ludwigshafen, Deutschland), das Ethylendiamintetraacetat (EDTA) enthält. Diese Komplexe sind stabil gegen Temperatur- (vorzugsweise bis mindestens 100 °C) und pH-Wert-Schwankungen. Die Komplexbildner fördern die Bildung von wasserlöslichen Komplexen mit Ionen. Die Komponente a6) dient insbesondere zur Einstellung des pH-Wertes sowie der Steuerung der Löslichkeit des Endprodukts

Sofern die Komponente a6) in den erfindungsgemäßen Kondensationsprodukten enthalten ist, beträgt das Verhältnis a1) zu a6) 10 bis 99 Gel.-% zu 1 bis 90 Gew.%, insbesondere 30 bis 80 Gew.-% zu 20 bis 70 Gew.-%.

In einer Ausführungsform der vorliegenden Erfindung wählt man die Komponenten a1) bis a6) in folgendem Verhältnis:
a1) im Bereich von insgesamt 10 bis 70 Gew.-% (Gewichtsprozent), bevorzugt insgesamt 20 bis 60 Gew.%, besonders bevorzugt insgesamt 35 bis 50 Gew.%,
a2) im Bereich von insgesamt 5 bis 40 Gew.-%, bevorzugt insgesamt 10 bis 30 Gew.-%, besonders bevorzugt insgesamt 15 bis 25 Gel.-%,
a3) im Bereich von insgesamt 5 bis 50 Gew.-%, bevorzugt insgesamt 10 bis 40 Gew.-%, besonders bevorzugt insgesamt 20 bis 30 Gew.-%, wobei Sulfonierungsmittel stets als SO₃ berechnet werden,
a4) im Bereich von 0 bis insgesamt 30 Gew.-%, bevorzugt insgesamt 10 bis 25 und besonders bevorzugt 15 bis 25 Gew.-%,
a5) im Bereich von insgesamt 10 bis 70 Gew.-%, bevorzugt insgesamt 20 bis 60 Gew.-%, besonders bevorzugt insgesamt 35 bis 50 Gew.-%,
   wobei Gew.-% jeweils auf die Summe aller Komponenten a1) und a2), gegebenenfalls a1) bis a5), bezogen sind,
a6) im Bereich von 0 bis Insgesamt 30 Gew.-%, bevorzugt bis insgesamt 25 Gew.% und besonders bevorzugt insgesamt bis 20 Gel.-%,
wobei die Gew.%-Angaben von a6) auf die Summe der Komponenten a1) und a2), gegebenenfalls a1) bis a5), bezogen sind.

Man kann beispielsweise bei Temperaturen im Bereich von 40 bis 200°C, bevorzugt 50 bis 110°C umsetzen. Üblicherweise passt man dabei die Temperatur der. Umsetzung an a1) und a2) an. Wünscht man beispielsweise aromatische Alkohole umzusetzen, so ist es bevorzugt, bei Temperaturen im Bereich von 50 bis 110°C umzusetzen. Natürlich ist es auch möglich, während der Umsetzung ein gewisses Temperaturprofil einzustellen. So ist es beispielsweise möglich, zunächst bei 90 bis 100°C die Reaktion zu starten und nach einiger Zeit, beispielsweise nach 2 bis 10 Stunden, auf 40 bis 75°C abzukühlen und die Umsetzung über einen Zeitraum von beispielsweise 1 bis 10 Stunden zu vervollständigen.

Man setzt beispielsweise bei Atmosphärendruck um, kann aber, falls es gewünscht ist, auch bei höheren Drücken, beispielsweise 1,1 bis 10 bar.

Durch die oben beschriebene Umsetzung erhält man Reaktionslösungen, die üblicherweise große Mengen an Säuren wie insbesondere Schwefelsäure, oder- im Falle des Einsatzes von Chlorsulfonsäure - HCl enthalten. Weiterhin können Reaktionslösungen große Mengen an Alkalimetallsulfat und/oder Alkalimetallchlorid enthalten.

Im Anschluss an die oben beschriebene Umsetzung kann man mit beispielsweise wässriger Alkalimetallhydroxidlösung oder wässrigem Ammoniak einen pH-Wert im Bereich von 3 bis 10, bevorzugt 3,5 bis 9 einstellen.

Durch Zugabe von Wasser zu durch die oben beschriebene Umsetzung erhältliche Reaktionslösungen kann man durch Verdünnen mit Wasser einen Wassergehalt im Bereich von 70 bis 95 Gew.-%, bevorzugt 75 bis 90 Gel.-% einstellen.

Aufgrund der in der Komponente a1) zwingend vorhandenen Carboxyl- und Hydroxyigruppen sowie in den einzelnen Komponenten eventuell vorhandenen weiteren sauren oder basischen Gruppen, umfasst die vorliegende Erfindung auch die entsprechend physiologisch oder toxikologisch verträgliche Salze der erfindungsgemäßen Kondensationsprodukte. Gegebenenfalls können die erfindungsgemäßen Kondensationsprodukte im Anschluss an deren Herstellung in physiologisch verträgliche Salze überführt werden.

Physiologisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein physiologisch verträgliches Anion oder Kation aufweisen. Geeignete physiologisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Theophyllinessigsäure, Methylen-bis-b-oxynaphthoe-, Benzolsulfon-, Benzoe-, Zitronen-, Ethansulfon-, Salicyl-, Fumar-, Glucon-, Glykol-, Isethion-, Milch-, Lactobion-, Malein-, Apfel-, Methansulfon-, Bernstein-, p-Toluolsulfon-, Wein- und Trifluoressigsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Die entsprechenden Salze der erfindungsgemäßen Kondensationsprodukte können durch herkömmliche Methoden, die dem Fachmann bekannt sind, erhalten werden, beispielsweise durch Umsetzung mit einer organischen oder anorganischen Säure oder Base in einem Lösungsmittel oder Dispergiermittel, oder durch Anionen- oder Kationenaustausch mit anderen Salzen.

Die vorliegende Erfindung schließt darüber hinaus alle Solvate der Kondensationsprodukte mit ein, beispielsweise Hydrate oder Addukte mit Alkohol, aktive Metaboliten sowie Derivate, die eine physiologisch annehmbare und abspaltbare Gruppe enthalten, beispielsweise Ester oder Amide.

Der hier verwendete Begriff "physiologisch funktionelles Derivat" bezeichnet jedes physiologisch verträgliche Derivat eines erfindungsgemäßen Kondensationsproduktes, z.B. einen Ester, der bei Verabreichung an einen Säuger, wie z.B. den Menschen, in der Lage ist, (direkt oder indirekt) ein erfindungsgemäßes Kondensationsprodukt oder einen aktiven Metaboliten hiervon zu bilden. Beispiele hierfür sind Acetyl-Phenoxyderivate, erhältlich durch Umsetzung von Essigsäureanhydrid und einer der vorhandenen Phenolgruppen.

Zu den physiologisch funktionellen Derivaten zählen auch Prodrugs der erfindungsgemäßen Kondensationsprodukte. Solche Prodrugs können in vivo zu einem erfindungsgemäßen Kondensationsprodukt metabolisiert werden. Diese Prodrugs können selbst wirksam sein oder nicht und sind ebenfalls Gegenstand der vorliegenden Erfindung.

Die erfindungsgemäßen Kondensationsprodukte können auch in verschiedenen polymorphen Formen vorliegen, z.B. als amorphe und kristalline polymorphe Formen. Alle polymorphen Formen der erfindungsgemäßen Kondensationsprodukte gehören in den Rahmen der Erfindung und sind ein weiterer Aspekt der Erfindung.

In einer weiteren Ausführungsform der vorliegenden Erfindung kann bei der Herstellung des erfindungsgemäßen Kondensationsproduktes nach Umsetzung der Komponenten a1) und a2) sowie gegebenenfalls a3) bis a5) ein molekülgrößenabhängiges Trennverfahren durchgeführt werden, vorzugsweise eine Ultrafiltration unter Erhalt einzelner Fraktionen des erfindungsgemäßen Kondensationsproduktes, beispielsweise einer niedermolekulare, einer mittelmolekulare sowie einer hochmolekulare Fraktion. Sinngemäßes gilt auch für die nachstehend beschriebenen Kondensationsprodukte (B). Die hochmolekulare Fraktion hat beispielsweise eine M_{w}-Wert ≥ 9000 g/mol (M_{w} = gewichtsmittleres Molekulargewicht), vorzugsweise einen M_{w}-Wert von 10.000 bis 100.000 g/mol. Die niedermolekulare Fraktion hat vorzugsweise einen M_{w}-Wert von 300 bis 3000 g/mol. Vorzugsweise beträgt in der hochmolekularen Fraktion das Verhältnis M_{w}/Mₙ < 10, insbesondere M_{w}/Mₙ < 5 (Mₙ = zahlenmittleres Molekulargewicht).

An molekülgrößenabhängigen Trennverfahren sind beispielsweise geeignet: Präparative Gelpermeationschromatographie und Membrantrennverfahren wie beispielsweise die Mikrofiltration, die Nanofiltration und insbesondere die Ultrafiltration. Auch Kombinationen von Mikrofiltration und Ultrafiltration sind geeignet. Mikrofiltrationen und Ultrafiltrationen und dafür erforderliche Membranen sind als solche bekannt und beschrieben beispielsweise in Ullmann's Encyclopedia of Industrial Chemistry, 6. Auflage, Bd. 21, Wiley-VCH Weinheim, S. 243 - 321. Nanofiltrationen und die dazu gehörigen Membranen sind als solche ebenfalls bekannt und beschrieben in R. Rautenbach, "Membranverfahren", Springer Verlag Berlin Heidelberg 1997.

Ultrafiltrationen sind als solche bekannt und werden im Allgemeinen als Querstromultrafiltrationen betrieben. Als Membranen sind handelsübliche Membranen geeignet, die beispielsweise aus organischen Materialien wie Polysulfonen oder Polyvinylidenfluorid oder bevorzugt aus anorganischen Materialien wie beispielsweise TiO₂, ZrO₂ oder Al₂O₃ hergestellt werden. Übliche Formen sind Kapillar-, Rohr- und Flachmembranen, letztere in Form von Membrankissen oder Spiralwickelmodulen.

Beispielsweise wendet man bei Membrantrennverfahren und insbesondere bei Ultrafiltrationen eine transmembrane Druckdifferenz, d.h. eine Druckdifferenz zwischen Feed und Permeat, im Bereich von 1 bis 200 bar, bevorzugt im Bereich von 1,2 bis 100 bar an.

In einer Ausführungsform liegt die Temperatur der nach Membrantrennverfahren behandelten Reaktionslösung im Bereich von 20 bis 70 °C, bevorzugt 25 bis 35 °C.

In einer Ausführungsform der vorliegenden Erfindung setzt man mindestens eine Membran mit einem molecular weight cut-off im Bereich von 1000 Dalton, bevorzugt 2000 Dalton, besonders bevorzugt 5000 Dalton, ganz besonders bevorzugt 7500 Dalton und noch mehr bevorzugt von 15.000 Dalton. Der molecular weight cut-off wird auch als Trenngrenze bezeichnet.

In einer Ausführungsform der vorliegenden Erfindung führt man die Ultrafiltration so durch, dass man ein bestimmtes Massenverhältnis von Permeat zu Retentat am Ende der Ultrafiltration einstellt. Die Retentatmenge bleibt üblicherweise bei der Ultrafiltration durch ständiges Nachdosieren von Wasser konstant, die Permeatmenge steigt im Laufe der Filtrationszeit an. Übliche Werte liegen im Bereich von 0,5:1 bis 10:1, bevorzugt 0,8:1 bis 5:1, besonders bevorzugt 1,0:1 bis 3:1.

Man erhält üblicherweise visuell im Wesentlichen transparente wässrige Lösungen von Kondensationsprodukten.

Es ist möglich, die Kondensationsprodukte aus den vorstehend beschriebenen Lösungen zu isolieren, beispielsweise durch Eindampfen des Wassers oder durch Sprühtrocknung.

In einer Ausführungsform der vorliegenden Erfindung haben die Kondensationsprodukte einen Salzgehalt an anorganischen Salzen wie beispielsweise Alkalimetallsulfat und Alkalimetallchlorid von 10 ppm bis weniger als 5 Gew.-%, bevorzugt weniger als 2 Gew.-%, besonders bevorzugt weniger als 1 Gew.% und ganz besonders bevorzugt weniger als 0,5 Gew.-%, bezogen auf das Trockengewicht von Kondensationsprodukt. Der Salzgehalt lässt sich beispielsweise durch Ionenchromatographie (IC) ermitteln, wie beispielsweise in Römpps Lexikon Chemie, 10. Auflage, Georg Thieme Verlag Stuttgart New York, Band 2, Stichwort: lonenchromatographie beschrieben.

In einer Ausführungsform der vorliegenden Erfindung haben die Kondensationsprodukte einen Restmonomergehalt von 10 ppm bis weniger als 5 Gew.-%, bevorzugt weniger als 2 Gew.%, bezogen auf das Trockengewicht des Kondensationsprodukts. Als Restmonomer werden im Rahmen der vorliegenden Erfindung nicht abreagierte Reaktanden a), c) und d) bezeichnet, die sich in Kondensationsprodukten befinden können. Der Restmonomergehalt lässt sich beispielsweise durch Gelpermeationschromatographie (GPC) oder vorzugsweise durch Ionenchromatographie (IC) oder Hochdruckflüssigkeitschromatographie (HPLC) bestimmen.

In einer Ausführungsform der vorliegenden Erfindung haben erfindungsgemäße Kondensationsprodukte einen Gehalt von freier Carbonylverbindung a2) einschließlich als Hydrat vorliegender Carbonylverbindung a2) im Bereich von 1 ppm bis weniger als 0,5 Gew.-%, bevorzugt 0,1 Gew.-% oder weniger, bezogen auf das Trockengewicht von erfindungsgemäßem Kondensationsprodukt. In dieser Ausführungsform bezieht sich die Menge an freier Carbonylverbindung a2) natürlich auf die Carbonylverbindung a2), das man bei der Umsetzung von a1) und a2) und gegebenenfalls a3) bis a5) eingesetzt hat. Hat man mehrere Carbonylverbindungen a2) eingesetzt, so bezieht sich der Gehalt an freier Carbonylverbindung a2) auf die Summe aller Carbonylverbindungen a2), die man bei der Umsetzung von a1) und a2) und gegebenenfalls a3) bis a5) eingesetzt hat. Die Bestimmung des Gehalts an freier Carbonylverbindung a2) kann man nach an sich bekannten Methoden durchführen. Ist Carbonylverbindung a2) bei Zimmertemperatur fest oder flüssig, so kann man den Gehalt an freier Carbonylverbindung a2) beispielsweise durch Gaschromatographie oder HPLC bestimmen. Handelt es sich bei Carbonylverbindung a2) um Formaldehyd, so lässt sich er sich beispielsweise photometrisch bestimmen. Eine besonders bevorzugte Methode zur Bestimmung von freiem Formaldehyd ist die Umsetzung mit Acetylaceton und Ammoniumacetat zu Diacetyldihydrolutidin und photometrische Messung von Diacetyldihydrolutidin bei einer Wellenlänge von 412 nm.

In einer Ausführungsform der vorliegenden Erfindung wird das vorstehend beschriebene Kondensationsprodukt als Gemisch mit mindestens einem (weiteren) Gerbstoff eingesetzt, insbesondere einem synthetischen oder pflanzlichen Gerbstoff. In einer Ausführungsform beträgt der M_{w}-Wert des weiteren Gerbstoffe ≤ 3000 g/mol, bevorzugt ist ein M_{w}-Wert zwischen 300 und 3000 g/mol.

Der weitere Gerbstoff kann sowohl ein anorganischer Gerbstoff, ein pflanzlicher Gerbstoff oder ein synthetischer Gerbstoff sein (siehe hierzu die vorstehend aufgeführte Definition gemäß Römpps Chemie Lexikon, 9. Auflage (1995), Georg Thieme Verlag, Stuttgart, Stichwort: "Gerbstoffe", Seiten 1541 bis 1542). Vorzugsweise werden als weitere Gerbstoffe pflanzliche oder synthetische Gerbstoffe verwendet, besonders bevorzugt sind hierbei synthetische Gerbstoffe. In einer Ausführungsform sind solche Gerbstoffe bevorzugt, die kein Formaldehyd als Edukt enthalten.

Der weitere Gerbstoff kann in beliebiger Konzentration dem erfindungsgemäßen Kondensationsprodukt zugegeben werden. Vorzugsweise liegt das erfindungsgemäße Kondensationsprodukt in einem solchen Gemisch zu mindestens 50 Gew.-% bezogen auf die Summe der Gerbstoffe vor.

Beispiele für pflanzliche Gerbstoffe sind Tannine wie Catechine oder Gallussäurederivate wie Gallate. Pflanzliche Gerbstoffe, die auf Gallussäurederivaten (wie Gallaten) beruhen, unterscheiden sich von den erfindungsgemäßen Kondensationsprodukten insbesondere darin, dass die Letztgenannten in ihren chemischen Strukturen (eine Vielzahl von) -CR¹R²-Brücken (Vernetzungen) aufweisen, die von der eingesetzten Carbonylverbindung a2) stammen und die in pflanzlichen Gerbstoffen nicht vorhanden sind. Sofern beispielsweise Formaldehyd als Komponente a2) eingesetzt wird, weisen die Kondensationsprodukte -CH₂-Brücken auf. Pflanzliche Gerbstoffe (Gallate) sind typischerweise oligomere Systeme, während es sich bei den Kondensationsprodukten gemäß der vorliegenden Erfindung vorzugsweise um Polymere handelt. In einer Ausführungsform haben die Kondensationsprodukte ein Molekulargewicht M_{w} ≥ 800 g/mol, vorzugsweise ≥ 2500 g/mol, insbesondere 10000 bis 50000 g/mol.

Bevorzugte pflanzliche Gerbstoffe sind Tannine aus der Gruppe der Catechine, Epicatechine und Epigallocatechine und deren Gallate.

Als Tannin versteht man prinzipiell natürlich vorkommende Polyphenole, wie sie beispielsweise in T. Okuda Phytochemistry, Band 66 (2005), Seiten 2012 bis 2031 oder Römpp's Chemielexikon, 9. Auflage (1995), Georg Thieme Verlag, Stuttgart, Stichwort "Tannine", Seiten 4452 bis 4453 aufgeführt sind. Bevorzugte Tannine sind Ellagitannine und Dehydroellagitannine, insbesondere Geraniin, Dehydrogeraniin, Furosinin, Ascorgeraniin, Geraniinsäure, Mallotusinsäure, Pentagalloylglukose, Camelliatannin A, Casuariin, Euphorbin E, Camelliatannin F, Agrimoniin, Trapanin B, Oenothein A, Oenothein B oder Gemin D , Lignin und Ligninsulfonate. Weiterhin bevorzugt sind Catechine, Epicatechine und Epigallocatechine.

Beispiele für ein geeignetes Catechin oder Derivate davon umfassen insbesondere Flavan-3-ole, Flavan-3,4-diole (Leukoanthocyanidine) sowie Flavanone, Flavone, Chalkone oder Dihydrocychalkone, Epicatechine und Epigallocatechine.

gallat, Methylgallat, Ethylgallat, n-Propylgallat, Isoamylgallat, Laurylgallat, Stearylgallat, Epigallocatechingallat sowie Gallussäure.

Beispielsweise lassen sich auch Extrakte des grünen Tees als pflanzliche Gerbstoffe einsetzen, ebenso Extrakte von Kastanien oder Mimosa.

Synthetische Gerbstoffe als solche sowie Verfahren zu deren Herstellung sind dem Fachmann bekannt. Geeignete synthetische Gerbstoffe, vorzugsweise mit einem M_{w}-Wert s 3000 g/mol, sind beispielsweise in EP-A 0 301 406 oder DE-A 10 2005 050 193.1 offenbart. Methoden, wie durch Steuerung der Syntheseparameter die Molmasse in einem bestimmten Bereich gesteuert werden kann, sind dem Fachmann bekannt.

Vorzugsweise enthalten die erfindungsgemäßen Gemische als synthetischen Gerbstoff mindestens eines der nachfolgend aufgeführten Kondensationsprodukte (B) bis (D).

### Kondensationsprodukt (B)

Kondensationsprodukt (B) ist erhältlich durch Umsetzung von
b1) mindestens einem Aromaten oder Heteroaromaten,
b2) mindestens einer Carbonylverbindung,
b3) mindestens einem Sulfonierungsmittel und
b4) mindestens einem Harnstoffderivat.

Die Komponenten b1) bis b4) entsprechen inklusive der bevorzugten Definitionen den Komponenten a2) bis a5) des erfindungsgemäßen Kondensationsproduktes, wobei die Komponente b1) der Komponente a5) entspricht. Weiterhin ist im Gegensatz zu Komponente a5) außer Phenol auch Dihydroxydiphenylsulfon, insbesondere 4,4'-Dihydroxydiphenylsulfon, eine besonders bevorzugt Komponente b1).

In einer Ausführungsform der vorliegenden Erfindung weisen die Kondensationsprodukte (B) einen M_{w}-Wert ≤ 3000 g/mol auf. Verfahren zur Herstellung von Kondensationsprodukten (B) mit einem niedrigen M_{w}-Wert (M_{w}-Wert ≤ 3000 g/mol) sind dem Fachmann bekannt. Solche Kondensationsprodukte lassen sich gezielt insbesondere durch Beeinflussung von Parametern wie Reaktionszeit, Temperatur (eher niedriger), die Wahl der Monomere (beeinflusst die Reaktivität, insbesondere Verwendung von Dihydroxydiphenylsulfonen oder pH-Wert (schwach sauer) herstellen. Alternativ können Kondensationsprodukte (B) auch dadurch hergestellt werden, dass - wie für das erfindungsgemäße Kondensationsprodukt beschrieben - im Anschluss an die Synthese eines entsprechenden Kondensationsproduktes ein molekülgrößenabhängiges Trennverfahren, vorzugsweise eine Ultrafiltration, durchgeführt wird, wobei das Kondensationsprodukt (B) von allen übrigen Bestandteilen isoliert wird. Kondensationsprodukte (B) mit dem gewünschten M_{w}-Wert können insbesondere durch Verwendung eines Membrans mit einem geeigneten Molecular weight cut-off-Bereich von 1000 D - 2500D abgetrennt und isoliert werden.

### Kondensationsprodukt (C)

Kondensationsprodukt (C) ist erhältlich durch Umsetzung von
c1) Melamin und/oder Harnstoff,
c2) Glyoxal, Glyoxylsäure oder einem Alkalisalz davon,
c3) mindestens einer aromatische Verbindung mit mindestens einer phenolischen Hydroxylgruppe und
c4) mindestens einer kondensierbaren Verbindung mit einer reaktiven stickstoffhaltigen Gruppe.

Die Kondensationsprodukte (C) als solche sowie Verfahren zu deren Herstellung sind dem Fachmann bekannt. Beispielsweise werden diese in EP-A 0 301 406 beschrieben und sind unter Bezugnahme in die vorliegende Erfindung mit ausgenommen.

Als Komponente c3) eignen sich beispielsweise Phenolsulfonsäure, Sulfosalicylsäure, Salicylsäure und 8-Hydroxychinolin oder 4,4'-Dihydroxydiphenylsulfon. Als Komponente c4) eignen sich Carbonsäureamide, Sulfonsäureamide, Imide, Harnstoffe, Amino- und Iminosäuren sowie Dialkylamine und Dialkanolamine. Beispiele dafür sind Acetamid, Benzoesäureamid, Formamid, Amidosulfonsäure, Succinimid, Glycin, Iminodiessigsäure, Phenylglycin, Harnstoff, Dicyandiamid, Diethanolamin oder Diethylamin. Saure Verbindungen können dabei in Form ihrer Alkalisalze einkondensiert werden. Besonders bevorzugt ist als Komponente c4) Acetamid und Amidosulfonsäure.

Ein bevorzugtes Kondensationsprodukt (C) ist erhältlich durch Umsetzung von
c1) Melamin und/oder Harnstoff,
c2) Glyoxal und/oder Glyoxylsäure sowie
c4) Amidosulfonsäure.

### Kondensationsprodukt (D)

Kondensationsprodukt (D) ist erhältlich durch Umsetzung von

### Kondensationsprodukt (D)

Kondensationsprodukt (D) ist erhältlich durch Umsetzung von
d1) mindestens einem cyclischen organischen Carbonat mit
d2) mindestens einer Verbindung mit mindestens zwei nucleophilen Gruppen pro Molekül, gewählt aus Sulfonsäure-, Hydroxyl-, primären oder sekundären Amino- oder Mercaptogruppen.

Kondensationsprodukte (D) als solche sowie Verfahren zu deren Herstellung sind dem Fachmann bekannt, sie sind beispielsweise in der deutschen Anmeldung mit der Nummer DE-A 10 2005 050 193.1 offenbart und unter Bezugnahme in die vorliegende Erfindung mit aufgenommen.

Unter cyclischen organischen Carbonaten (Komponente d1) versteht man im Rahmen der vorliegenden Erfindung organische Kohlensäureester, die mindestens eine cyclische Gruppe aufweisen.

Bevorzugt handelt es sich bei cyclischen organischen Carbonaten um solche organischen Kohlensäureester, bei denen die Kohlensäureestergruppe Bestandteil eines cyclischen Systems ist.

In einer Ausführungsform der vorliegenden Erfindung wählt man cyclisches organisches Carbonat (d1) aus Verbindungen der allgemeinen Formel (II) 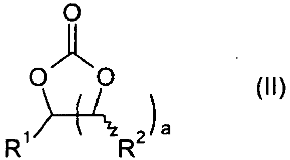 wobei die Variablen wie folgt definiert sind:
- R¹: gewählt aus C₁-C₄-Alkyl, verzweigt oder vorzugsweise linear, beispielsweise Methyl, Ethyl, n-Propyl, iso-Propyl, n-Butyl, iso-Butyl, sec-Butyl, tert.-Butyl, ganz bevorzugt Methyl und Ethyl, und ganz besonders bevorzugt Wasserstoff,
- R²: gegebenenfalls verschieden oder vorzugsweise gleich und unabhängig voneinander gewählt aus Wasserstoff und C₁-C₄-Alkyl, verzweigt oder vorzugsweise linear, beispielsweise Methyl, Ethyl, n-Propyl, iso-Propyl, n-Butyl, iso-Butyl, sec-Butyl, tert.-Butyl, ganz bevorzugt Methyl und Ethyl, und ganz besonders bevorzugt jeweils gleich und Wasserstoff,
- a: eine ganze Zahl im Bereich von 1 bis 3, bevorzugt 2 und besonders bevorzugt 1.

Besonders bevorzugte cyclische organische Carbonate d1) sind Propylencarbonat oder Ethylencarbonat. Mischungen von Propylencarbonat (R¹ = Methyl, R² = Wasserstoff, a = 1) und Ethylencarbonat (R¹ = R² = Wasserstoff, a = 1), insbesondere bei Zimmertemperatur flüssige Mischungen von Propylencarbonat und Ethylencarbonat sind ebenfalls besonders bevorzugt.

Unter Komponente d2) werden solche Verbindungen verstanden, die zwei zu nucleophilen Reaktionen befähigte Gruppen wie beispielsweise Sulfonsäuregruppen, Hydroxylgruppen, Mercaptogruppen oder primäre oder sekundäre Aminogruppen aufweisen.

Beispiele für geeignete Verbindungen d2) können aufweisen:
mindestens zwei nucleophile Hydroxylgruppen pro Molekül,
mindestens zwei nucleophile Mercaptogruppen pro Molekül,
mindestens zwei nucleophile primäre oder sekundäre Aminogruppen pro Molekül, beispielsweise zwei oder drei nucleophile primäre oder sekundäre Aminogruppen pro Molekül,
mindestens eine nucleophile Hydroxylgruppe oder Mercaptogruppe und mindestens eine nucleophile primäre oder sekundäre Aminogruppe pro Molekül oder
mindestens eine nucleophile Hydroxylgruppe und mindestens eine nucleophile Mercaptogruppe pro Molekül,
mindestens eine nucleophile Hydroxylgruppe oder primäre oder sekundäre Aminogruppe und eine Sulfonsäuregruppe pro Molekül.

Schwefelsäure ist keine Verbindung d2) im Sinne der vorliegenden Erfindung.

Beispiele für nucleophile Hydroxylgruppen sind OH-Gruppen von primären und sekundären Alkoholen und insbesondere phenolische OH-Gruppen.

Beispiele für nucleophile Mercaptogruppen sind SH-Gruppen, aliphatisch oder aromatisch.

Beispiele für nucleophile Aminogruppen sind -NHR³-Gruppen, aliphatisch oder aromatisch, wobei R³ gewählt wird aus Wasserstoff, C₁-C₄-Alkyl, wie vorstehend definiert, und CN, oder die NH₂-Gruppe von beispielsweise Amidosulfonsäure.

OH-Gruppen und NH-Gruppen, die Bestandteile von Aminalgruppen, Halbaminalgruppen oder Hydratgruppen von Ketonen oder Aldehyden sind, sind keine nucleophilen Hydroxylgruppen beziehungsweise Aminogruppen im Sinne der vorliegenden Erfindung. Auch OH-Gruppen und NH-Gruppen, die Bestandteile von Carbonsäuregruppen oder Carbonsäureamidgruppen sind, sind keine nucleophilen Hydroxylgruppen beziehungsweise Aminogruppen im Sinne der vorliegenden Erfindung.

Bevorzugte Beispiele für Verbindungen d2) sind
i) Harnstoffe, unsubstituiert oder ein- oder zweifach N,N'-substituiert mit C₁-C₄-Alkyl, Biuret, insbesondere unsubstituierter Harnstoff,
ii) heterocyclische Verbindungen mit mindestens zwei NH₂-Gruppen pro Molekül, beispielsweise Adenin und insbesondere Melamin,
iii) Benzoguanamin, Dicyandiamid, Guanidin,
iv) Verbindungen der allgemeinen Formel (III) 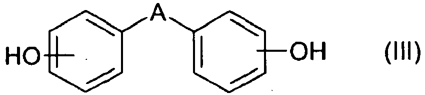 in denen A eine bivalente Gruppe bedeutet, beispielsweise -CH₂-, -CH₂CH₂-, -CH(CH₃)-, -C(CH₃)₂-, -CO-, -SO₂-, bevorzugt 4,4'-Dihydroxybiphenyl, 2,4'-Dihydroxydiphenylsulfon, besonders bevorzugt 4,4'-Dihydroxydiphenylsulfon, Mischungen von 4,4'-Dihydroxydiphenylsulfon und 2,4'-Dihydroxydiphenylsulfon, beispielsweise in einem Gewichtsverhältnis von 8 : 1 bis 8 : 1,5, sowie Bisphenol A.

Weitere bevorzugte Beispiele für Verbindung d2) sind 4-Hydroxyphenylsulfonsäure und Amidosulfonsäure.

Besonders bevorzugte Verbindungen d2) sind ausgewählt aus Melamin, Biuret, Dicyanamid, Amidosulfonsäure und 4,4'Dihydroxydiphenylsulfon.

In einer Ausführungsform der vorliegenden Erfindung werden Gemische eingesetzt, in denen mindestens ein erfindungsgemäßes Kondensationsprodukt und/oder mindestens ein synthetischer Gerbstoff mit einem M_{w}-Wert ≤ 3000 g/mol hergestellt werden unter Verwendung von mindestens einer Verbindung, die mindestens eine Hydroxylgruppe enthält beziehungsweise mit einer solchen Gruppe substituiert ist. Vorzugsweise wird dies erzielt, indem die Komponente c3) vorhanden ist und/oder
die Komponente d2) mindestens eine Verbindung enthält mit mindestens einer Hydroxylgruppe als nucleophiler Gruppe.

In einer weiteren bevorzugten Ausführungsform der vorliegenden Erfindung werden Gemische eingesetzt, bei denen der weitere Gerbstoff formaldehydfrei ist, vorzugsweise ein synthetischer formaldehydfreier Gerbstoff. Vorzugsweise wird dies erzielt, indem ein Kondensationsprodukt (C) oder Kondensationsprodukt (D) im Gemisch eingesetzt wird.

Die erfindungsgemäßen Kondensationsprodukte eignen sich insbesondere als antivirales Agens, also als Arzneimittel gegen Viren, auch als Vrustatika oder virucide Mittel bezeichnet. Vorzugsweise eignen sie sich als antivirales Agens gegenüber humanen Papillomviren, speziell Typ 16, 18, 6 und 11, endogenen Retroviren, insbesondere HERV-Typ (Humane endogene Retroviren), Herpes-Viren, insbesondere HSV-1, HCMV-Viren (Humanes Zytomegalievirus) oder HIV-Viren.

Weiterhin eignen sich die erfindungsgemäßen Kondensationsprodukte vorzugsweise als antivirales Agens gegenüber Coronaviren (z.B. SARS (Severe Acute Respiratory Syndrome)-assoziiertes Coronavirus), Flaviviren (z.B. West-Nil-Virus (WNV)), Togaviren (zB. Chikungunyavirus) oder Paramoxyviren (z.B. Masern, Respiratorisches Syncytialvirus (RSV).

Vorzugsweise eignen sich die erfindungsgemäßen Kondensationsprodukte zur Herstellung von einem Medikament zur Prophylaxe und/oder Behandlung von Genitalwarzen, Gebärmutterhalskrebs, allergischen oder nicht-allergischen Ekzemen, insbesondere Neurodermitis (endogenes Ekzem), Wundsein, Juckreiz, entzündlichen Erkrankungen, Autoimmunerkrankungen, insbesondere Arthritis, Rheuma, von melanomen Karzinomen, Entzündungen der Haut, Herpes, insbesondere Herpes labilis und Herpes simplex, Windpocken, Gürtelrose, Influenza oder Aids (HIV).

In einer Ausführungsform der vorliegenden Erfindung handelt es sich bei Arzneimitteln um Arzneimittel zur lokalen Behandlung von allergischen oder nicht-allergischen Ekzemen, Wundsein oder Juckreiz. Vorzugsweise wird in dieser Ausführungsform Neurodermitis (endogenes Ekzem) behandelt.

In einer speziellen Ausführungsform der vorliegenden Erfindung handelt es sich bei Arzneimitteln um Arzneimittel zur Behandlung von entzündlichen Erkrankungen der Haut, bei denen es durch enzymatische Aktivität z.B. der humanen Leukozyten-Elastase zur Bildung von Bläschen, Pusteln und zur so genannten Spongiose in der Oberhaut kommt. Die Arzneimittel werden bevorzugt äußerlich appliziert

In einer bevorzugten Ausführungsform der vorliegenden Erfindung handelt es sich bei Arzneimitteln um Arzneimittel gegen Viren, vorzugsweise Retroviren, beispielsweise RNS-Viren (Ribonucleinsäure-Viren) und DNS-Viren (Desoxyribonucleinsäure-Viren) und insbesondere Herpes-Viren, beispielsweise Viren, die Herpes simplex (HS-Viren) erzeugen, oder auch um Viren, die Windpocken und Influenza erzeugen. Ferner ist anzumerken, dass die erfindungsgemäßen Wirkstoffe sowohl gegen hydrophile als auch ebenso wirksam gegen lipophile/hydrophobe Viren eingesetzt werden können.

In einer weiteren Ausführungsform der vorliegenden Erfindung handelt es sich bei Arzneimitteln um Arzneimittel gegen HIV-Viren (Human Imuno Deficiency Virus). Der HIV-Virus ist dafür bekannt, dass er Aids (Aquired Imuno Deficiency Syndrom) verursacht.

In einer weiteren bevorzugten Ausführungsform der vorliegenden Erfindung handelt es sich bei Arzneimitteln um Arzneimittel gegen Humane Papillomviren sowie von endogenen Retroviren (HERV-Typ). Bei den Human-Papillomviren handelt es sich insbesondere um den Typ 16, 18, 6 und 11. In dieser Hinsicht eignen sich die erfindungsgemäßen Kondensationsprodukte insbesondere zur äußerlichen Medikation von Genitalwarzen und Gebärmutterhalskrebs. Im Zusammenhang mit der Behandlung von HERV-Viren (insbesondere HERV-K) eignen sich die erfindungsgemäßen Kondensationsprodukt zur Behandlung von Autoimmunkrankheiten (Arthritis) sowie präventiv gegen melanome Karzinome.

In den vorstehenden Ausführungen umfasst der Begriff Behandlung auch die Prophylaxe, Therapie oder Heilung der vorgenannten Krankheiten.

Die erfindungsgemäßen Kondensationsprodukte können Tieren und Menschen, bevorzugt Säugetieren und Menschen, besonders bevorzugt Menschen, verabreicht werden. Die erfindungsgemäßen Kondensationsprodukte können dabei selbst als Arzneimittel, in Mischungen miteinander oder Mischungen mit anderen Arzneimitteln oder in Form von pharmazeutischen Zusammensetzungen verabreicht werden. Folglich sind die Verwendung von den erfindungsgemäßen Kondensationsprodukten zur Herstellung eines oder mehrerer Medikamente zur Prophylaxe und/oder Behandlung der vorgenannten Krankheiten oder als antivirales Agens, pharmazeutische Zusammensetzungen enthaltend eine wirksame Menge von mindestens einem erfindungsgemäßen Kondensationsprodukt sowie der Verwendung dieser pharmazeutischen Zusammensetzungen zur Prophylaxe und/oder Behandlung der vorgenannten Krankheiten ebenfalls Gegenstand der vorliegenden Erfindung.

Die erfindungsgemäßen pharmazeutischen Zusammensetzungen umfassen eine wirksame Menge von mindestens einem erfindungsgemäßen Kondensationsprodukt sowie einen physiologisch verträglichen Träger. Die pharmazeutischen Zusammensetzungen können dabei in unterschiedlichen Darreichungsformen vorliegen, insbesondere in Form einer Pille, Tablette, Lutschtablette, Granulat, Kapsel, harte oder weiche Gelatinekapsel, wässrigen Lösung, alkoholischen Lösung, ölartigen Lösung, Sirup, Emulsion, Suspension, Zäpfchen, Pastille, Lösung zur Injektion oder Infusion, Salbe, Tinktur, Creme, Lotion, Puder, Spray, eines transdermalen therapeutischen Systems, Nasenspray, Aerosol, Aerosol-Mischung, Mikrokapsel, Implantat, Stab, Pflaster oder Gel. Ebenso kann die erfindungsgemäße pharmazeutische Zusammensetzung auch Bestandteil von Health-Care Produkten wie Sonnenschutzcremes, Nasensprays, Mundwässern, Zahnpasten, Pflastern, (Feucht)-Tüchern, Waschlotionen oder Shampoos sein.

Je nach verwendeter Darreichungsform werden die erfindungsgemäßen Kondensationsprodukte mit physiologisch verträglichen Trägern, die dem Fachmann als solche bekannt sind, zu den efindungsgemäßen pharmazeutischen Zusammensetzungen verarbeitet. Der Träger muss natürlich verträglich sein in dem Sinn, dass er mit den anderen Bestandteilen der Zusammensetzung kompatibel ist und nicht gesundheitsschädlich für den Patienten ist (physiologisch verträglich). Der Träger kann ein Feststoff oder eine Flüssigkeit oder beides sein und wird vorzugsweise mit der Verbindung als Einzeldosis formuliert, beispielsweise als Tablette, die von 0,05 bis 95 Gew.-% des Wirkstoffs (erfindungsgemäßes Kondensationsprodukt) enthalten kann. Weitere pharmazeutisch aktive Substanzen können ebenfalls vorhanden sein. Die erfindungsgemäßen pharmazeutischen Zusammensetzungen können nach einer der bekannten pharmazeutischen Methoden hergestellt werden, die im Wesentlichen darin besteht, dass die Bestandteile mit pharmakologisch verträglichen Trägern und/oder weiteren Hilfsstoffen wie Füllstoffe, Bindemittel, Gleitmittel, Netzmittel, Stabilisatoren und so weiter gemischt werden.

Bevorzugte pharmazeutische Zusammensetzungen im Rahmen der vorliegenden Erfindung sind nachfolgend aufgeführt.

In einer Ausführungsform der vorliegenden Erfindung können erfindungsgemäße Salben, Cremes, Fettcremes, Gele, Lotionen beziehungsweise Puder jeweils im Bereich von 0,1 bis 5 Gew.%, bevorzugt 0,2 bis 3 Gew.% erfindungsgemäße Kondensationsprodukte enthalten, bezogen auf die jeweilige Salbe, Creme, Fettcreme, Lotion beziehungsweise das jeweilige Gel oder Puder.

In einer Ausführungsform der vorliegenden Erfindung können erfindungsgemäße Pulver beziehungsweise Konzentrate im Bereich von 1 bis 75 Gew.-%, bevorzugt 10 bis 65 Gew.-% erfindungsgemäßes Kondensationsprodukt enthalten, bezogen auf das jeweilige Pulver beziehungsweise Konzentrat.

Erfindungsgemäße Cremes sind üblicherweise Öl-in-Wasser-Emulsionen, erfindungsgemäße Salben sind üblicherweise Wasser-in-Öl-Emulsionen. Erfindungsgemäße Salben und Cremes enthalten neben vorzugsweise gereinigtem Wasser eine oder mehrere Ölkomponenten und vorzugsweise eine oder mehrere oberflächenaktive Substanzen, beispielsweise einen oder mehrere Emulgatoren oder Schutzkolloide. Weiterhin können erfindungsgemäße Salben und Fettcremes - wie andere erfindungsgemäße Darreichungsformen der erfindungsgemäßen Kondensationsprodukte auch - Konservierungsmittel enthalten wie beispielsweise Sorbinsäure.

Geeignete Ölkomponenten sind natürliche und synthetische Wachse, natürliche und synthetische Öle wie beispielsweise Nussöl, Fischöl, Olivenöl und Polymere wie beispielsweise Polyacrylsäure, Polydimethylsiloxan und Polymethylphenylsiloxan.

Geeignete oberflächenaktive Substanzen sind beispielsweise Verbindungen der allgemeinen Formel (IV)

CH₃-(CH₂)ₙ-X-R³ (IV)

wobei die Variablen wie folgt definiert sind:
- n: ist eine ganze Zahl im Bereich von 0 bis 20, bevorzugt eine gerade Zahl im Bereich von 2 bis 16 bedeutet und
- X: steht für zweibindige Gruppen, die mindestens ein von Kohlenstoff und Wasserstoff verschiedenes Atom, bevorzugt Stickstoff und besonders bevorzugt Sauerstoff tragen, insbesondere -O- und -COO-,
- R³: wird gewählt aus
Wasserstoff,

C₁-C₁₀-Alkylgruppen wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; besonders bevorzugt C₁-C₄-Alkyl wie Methyl, Ethyl, n-Propyl, iso-Propyl, n-Butyl, iso-Butyl, sec.-Butyl und tert.-Butyl,

-(CH₂-CH₂-O)ₘ-H, wobei m eine ganze Zahl im Bereich von 1 bis 100, bevorzugt bis 25 ist,

CH₃-(CH₂)ₙ-X-(O-CH₂-CH₂)ₘ-, wobei X und n jeweils verschieden oder vorzugsweise gleich sein können.

Weiterhin können erfindungsgemäße Salben und Cremes - wie andere erfindungsgemäße Darreichungsformen der erfindungsgemäßen Kondensationsprodukte auch - organische Lösungsmittel enthalten wie beispielsweise Propylenglykol und Glycerin.

Bevorzugte Beispiele für oberflächenaktive Substanzen sind beispielsweise Isopropyltetradecanoat, Cetylalkohol, Palmitinsäure, Stearinsäure, Polyoxyethylen-2-stearylether, α-n-Dodecyl-ω-hydroxypolyoxyethylen mit im Mittel 10 Ethylenoxideinheiten, 2-Phenoxyethanol, Polyoxyethylen-21-stearylether.

Erfindungsgemäße Fettcremes sind üblicherweise Wasser-in-Öl-Emulsionen und enthalten neben vorzugsweise gereinigtem Wasser ein oder mehrere Ölkomponenten und vorzugsweise eine oder mehrere oberflächenaktive Substanzen, beispielsweise einen oder mehrere Emulgatoren oder Schutzkolloide.

Geeignete Ölkomponenten sind neben den vorstehend beschriebenen Ölkomponenten natürliche und synthetische Fette wie beispielsweise ein- oder mehrfach ethylenisch ungesättigte Fettsäureglyceride.

Weiterhin können erfindungsgemäße Fettcremes eine oder mehrere der folgenden Substanzen enthalten: Methyl-4-hydroxybenzoat, Propyl-4-hydroxybenzoat, wässrige Sorbit-Lösung, Tris[n-dodecylpoly(oxoethylen)-4]phosphat, Cetylstearylalkohol, Hexyllaurat, Vitamin-F-Glycerolester, Dimeticon 350, Calciumlactat-Pentahydrat. Erfindungsgemäße Gele können beispielsweise Polyacrylsäure, Natriumhydroxid und Butylhydroxyanisol, beispielsweise 4-Methoxy-2-tert.-butylphenol, 4-Methoxy-3-tert.-butylphenol und Mischungen der beiden vorgenannten Verbindungen enthalten.

Erfindungsgemäße Lotionen können beispielsweise mindestens einen der im Folgenden genannten Stoffe enthalten: Glycerin, Zinkoxid, Talkum, Lecithin, hochdisperses Siliciumdioxid, Isopropanol, Methyl(4-hydroxybenzoat), Carageenan, Natriumsalz und Phosphorsäureester der allgemeinen Formel (V) 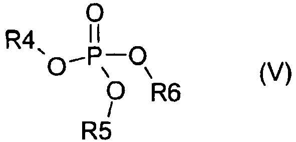 in denen R⁴, R⁵ und R⁶ gleich oder verschieden sein können und gewählt werden aus n-C₁₀-C₂₀-Alkyl, insbesondere n-C₁₆-C₁₈-Alkyl und H-(O-CH₂-CH₂)ₘ, wobei m wie oben stehend definiert ist.

Erfindungsgemäße Puder können beispielsweise enthalten: Calciumlactat-Pentahydrat, Talkum, Maisstärke, 2-n-Octyl-1-dodecanol, Siliciumdioxid.

Erfindungsgemäße Pulver zur Herstellung von Gebrauchslösungen können beispielsweise Calciumlactat 5 H₂O und Natriumsulfat (als Trägermaterial) enthalten.

Erfindungsgemäße Konzentrate zur Herstellung von Gebrauchslösungen können beispielsweise enthalten: Natriumsalz des Dodecylpoly(oxyethylen)-2-hydrogensulfats, Natriumsulfat als Trägermaterial.

Anstatt erfindungsgemäße Salben, Cremes, Fettcremes, Gele, Lotionen, Puder, Pulver oder Konzentrate auf ihre Wirksamkeit zu untersuchen, kann man erfindungsgemäße Kondensationsprodukte, gegebenenfalls als Stammlösung, auf ihre Wirksamkeit untersuchen. Geeignete Untersuchungsmethoden sind Untersuchungen auf Hemmung von ausgesuchten Enzymen, beispielsweise humane Leukozyten-Elastase oder der Protease Plasmin. Weiterhin kann man untersuchen, in welchem Maße die Replikation betreffender Viren gehemmt wird. Solche Untersuchungsmethoden werden im nachfolgend Text (Pharmakalogische Untersuchungen) noch genauer beschrieben.

Ein weiterer Gegenstand der vorliegenden Erfindung ist die Verwendung eines erfindungsgemäßen Kondensationsproduktes zur Desinfektion, als Desinfektionsmittel oder Bestandteil eines Desinfektionsmittels. Insbesondere finden die erfindungsgemäßen Kondensationsprodukte Verwendung im Bereich von Krankenhäusern, insbesondere Krankenhaus-Intensivstationen, Toiletten, Waschräumen, Haushalten, der Lebensmittelproduktion oder in Stallungen oder Käfigen von Tieren, insbesondere von Vögeln, Schweinen und Rindern.

Die erfindungsgemäßen Kondensationsprodukte zeichnen sich bei ihrer Verwendung als Desinfektionsmittel dadurch aus, dass sie eine überraschend gute Breitbandwirkung gegenüber Pilzen, Bakterien und Viren haben sowie eine geringere Toxizität gegenüber den üblichen Mitteln beziehungsweise Mischungen, die als Desinfektionsmittel gemäß des Standes der Technik verwendet werden, aufweisen. Weiterhin sind sie weder flüchtig noch schleimhautreizend und sie sind sowohl als flüssige oder auch streufähige Pulvereinstellung leicht herstellbar. Insbesondere eignen sich die efindungsgemäßen Kondensationsprodukte zur Anwendung in Stallungen oder Käfigen von Tieren, vorzugsweise auf Stroh.

Ein weiterer Gegenstand der vorliegenden Erfindung ist somit auch ein Desinfektionsmittel enthaltend mindestens ein erfindungsgemäßes Kondensationsprodukt erhältlich durch Umsetzung von
a1) mindestens einem Aromaten oder Heteroaromaten, wobei der Aromat oder Heteroaromat mit mindestens einer Hydroxy- (-OH) und mindestens einer Carboxy-Gruppe (-COOH) substituiert ist, und wobei die Hydroxy- und/oder Carboxy-Gruppe in Salzform vorliegen können,
a2) mindestens einer Carbonylverbindung,
a3) mindestens einem Sulfonierungsmittel
a4) mindestens einem Hamstoffderivat, und
a5) mindestens einen weiteren Aromaten oder Heteroaromaten, ausgewählt aus Benzol, Naphthalin, Anthracen, aromatischen Alkoholen, aromatischen Ethern und aromatischen Sulfonen, wobei die vorgenannten Verbindungen substituiert sein können und diese Verbindungen nicht mit mindestens einer Hydroxy- (-OH) und mindestens einer Carboxy-Gruppe (-COOH), oder die Salzform dieser Verbindungen, substituiert sind,
oder ein physiologisch verträgliches Salz davon,
wobei die Umsetzung in einer oder in mehreren Stufen erfolgen kann, wobei das Umsetzungsprodukt der Komponenten a1) und a3)
ohne vorherige Isolierung mit Komponente a2), Komponente a4) und Komponente a5) oder
mit vorheriger Isolierung mit dem Umsetzungsprodukt von Komponenten a2), a4) und a5)
umgesetzt wird,
unter der Voraussetzung, dass die Komponente a1) nicht Salicylsäure oder Sulfosalicylsäure ist, wenn die Komponente a4) Melamin ist.

Die erfindungsgemäßen Desinfektionsmittel sind somit nicht zur Verabreichung als Arzneimittel gedacht, sondern sie sind zur Desinfektion von beispielsweise den vorstehend aufgeführten Gegenständen geeignet. In den erfindungsgemäßen Desinfektionsmitteln ist mindestens ein erfindungsgemäßes Kondensationsprodukt in den üblichen Konzentrationen enthalten. Weitere Komponenten, die in den erfindungsgemäßen Desinfektionsmitteln enthalten sind, sind dem Fachmann bekannt. Solche Komponenten können je nach Anwendungsgebiet variieren, gleiches gilt für die Konzentration an dem erfindungsgemäßen Kondensationsprodukt.

Ein weiterer Gegenstand der vorliegenden Erfindung ist die Verwendung der erfindungsgemäßen Kondensationsprodukte als Gerbstoff bzw. als Gerbereihilfsmittel. Vorzugsweise eignen sich die erfindungsgemäßen Kondensationsprodukte zum Gerben von Ledern und/oder Häuten. Verfahren zum Gerben (Gerbverfahren) als solche sind dem Fachmann bekannt, sie beispielsweise EP-A 0 301 406.

Die Erfindung soll an den nachfolgenden Beispielen verdeutlicht werden.

### Beispiele

### Allgemeine Angaben

ppm beziehen sich stets auf Gewichtsanteile, sofern nicht anders angegeben.

Die Molekulargewichtsbestimmungen werden mit Gelpermeationschromatographie (GPC) durchgeführt:
Stationäre Phase: mit Ethylenglykoldimethacrylat vernetztes Poly-(2-hydroxymeth-acrylat)-Gel, kommerziell erhältlich als HEMA BIO von Fa. PSS, Mainz, Deutschland.
Laufmittel: Gemisch aus 30 Gew.-% Tetrahydrofuran (THF), 10 Gew.-% Acrylnitril, 60 Gew.-% 1-molare NaNO₃-Lösung
Interner Standard: 0,001 Gew.-% Benzophenon, bezogen auf Laufmittel
Fluss: 1,5 ml/min
Konzentration: 1 Gew.-% im Laufmittel mit internem Standard
Detektion: UV/Vis-spektrometrisch bei 254 nm
Kalibrierung mit Polystyrol-Eichteils der Fa. PSS.
Mₙ: zahlenmittleres Molekulargewicht in [g/mol]
M_{w}: gewichtsmitteleres Molekulargewicht in [g/mol]

Zur Bestimmung von freiem Formaldehyd wird eine Flow-Injection-Apparatur nach Huber eingesetzt, s. Fresenius Z. Anal. Chem. 1981, 309, 389. Als Säule wählt man eine thermostatisierte Reaktionssäule 170 x 10 mm, gefüllt mit Glaskügelchen, die bei 75°C betrieben werden. Der Detektor (Durchflussdetektor) wird auf eine Wellenlänge von 412 nm eingestellt. Man geht wie folgt vor:

Zur Herstellung einer Reagenzlösung löst man 62,5 g Ammoniumacetat in 500 ml destilliertem Wasser, gibt 7,5 ml konzentrierte Essigsäure und 5,0 ml Acetylaceton zu und füllt mit destilliertem Wasser auf 1000 ml auf.

In einen 10-ml-Messkolben wiegt man 0,1 g des zu untersuchenden Kondensationsprodukts ein, füllt mit destilliertem Wasser auf 10 ml auf und erhält die jeweilige Probelösung.

Man gibt jeweils 100 µl Probelösung auf, mischt sie mit Reagenzlösung und stellt eine mittlere Verweilzeit von 1,5 Minuten ein, was einem Fluss von 35 ml/min entspricht.

Zur Ermittlung der absoluten Werte wird die Flow-Injection-Apparatur mit FormaldehydLösungen mit bekanntem Gehalt kalibriert.

### 1. Synthesebeispiele von Kondensationsprodukten

### Beispiel 1

### Reaktanden:

a) Phenol,
b) konzentrierte Schwefelsäure,
c) Harnstoff
d) Formaldehyd
e) Gallussäure

### Vorgehen:

47,1 g Phenol werden in einer Rührapparatur vorgelegt und während 25 Minuten mit 57,1 g konzentrierter Schwefelsäure (96 Gew.-%) versetzt. Dabei wird darauf geachtet, dass die Temperatur 105°C nicht übersteigt. Anschließend wird das Reaktionsgemisch 2 Stunden bei 100 bis 105°C gerührt. Das Reaktionsgemisch wird dann mit 7,9 g Wasser verdünnt. Es werden 64,6 g wässrige Harnstofflösung (50 Gew.-%) zudosiert, wobei die Temperatur auf 95°C steigt; anschließend wird auf 75°C gekühlt. Über einen Zeitraum von 90 Minuten werden 94,6 g wässrige Formaldehydlösung (30 Gew.-%) zugefügt, wobei darauf geachtet wird, dass die Temperatur nicht über 75°C steigt. Anschließend wird mit 17,3 g wässriger Natronlauge (50 Gew.-%) teilneutralisiert, und 6,9 g Wasser wird zugefügt. Bei einer Temperatur von 50 bis 55°C werden 56,5 g Gallussäure und 0,6 g eines Komplexbildners auf Basis Etyhlendiamin - Tetraessigsäure zugegeben. Das Reaktionsgemisch wird dann 30 Minuten bei 55°C nachgerührt, bevor 26,3 g wässrige Formaldehydlösung (30 Gew.-%) innerhalb von 15 bis 20 Minuten bei 55 bis 60°C zudosiert werden. 74,4 g wässrige Natronlauge (50 Gew.-%) und 46,2 g Wasser werden zugegeben. Die Endeinstellung vom pH-Wert bis pH-Wert 7,0 bis 7,4 erfolgt durch Zugabe von 16,4 g Schwefelsäure (50 Gew.-%). Nach Verdampfung der flüchtigen Anteile erhält man 465 g Kondensationsprodukt 1 als Feststoff mit tieflila Farbe.

Die Analyse des Kondensationsproduktes 1 ergibt folgende Werte:
Phenol durch HPLC: < 0,1 Gew.-%;
4-Phenolsulfonsäure durch HPLC: 9,8 Gew.-%;
freier Formaldehyd: < 20 ppm;
Mₙ 1740 g/mol, M_{w} 9140 g/mol, bestimmt durch GPC.

### Vergleichsbeispiel 2

20,4 g Phenol werden in einer Rührapparatur vorgelegt und während 25 Minuten mit 24,7 g konzentrierter Schwefelsäure (96 Gew.-%) versetzt. Dabei wird darauf geachtet, dass die Temperatur 105°C nicht übersteigt. Anschließend wird das Reaktionsgemisch 2 Stunden bei 100 bis 105°C gerührt. Das Reaktionsgemisch wird dann mit 3,4 g Wasser verdünnt. Es werden 28,1 g wässrige Harnstofflösung (50 Gew.-%) zudosiert, wobei die Temperatur auf 95°C steigt; anschließend wird auf 75°C gekühlt. Über einen Zeitraum von 90 Minuten werden 41,0 g wässrige Formaldehydlösung (30 Gew.-%) zugefügt, wobei darauf geachtet wird, dass die Temperatur nicht über 75°C steigt. Anschließend wird mit 7,5 g wässriger Natronlauge (50 Gew.-%) teilneutralisiert, und 3 g Wasser wird zugefügt. Bei einer Temperatur von 50 bis 55°C werden 13,6 g Phenol und 0,3 g eines Komplexbildners auf Basis Etyhlendiamin - Tetraessigsäure zugegeben. Das Reaktionsgemisch wird dann 30 Minuten bei 55°C nachgerührt, bevor 11,4 g wässrige Formaldehydlösung (30 Gew.-%) innerhalb von 15 bis 20 Minuten bei 55 bis 60°C zudosiert werden. 32,2 g wässrige Natronlauge (50 Gew.-%) und 20 g Wasser werden zugegeben. Die Endeinstellung vom pH-Wert bis pH 7,0 - 7,4 erfolgt durch Zugabe von 8,2 g Schwefelsäure (50 Gew.-%). Nach Verdampfung der flüchtigen Anteile erhält man 200 g Kondensationsprodukt 2 als farblosen Feststoff.

Die Analyse des Kondensationsproduktes 2 ergibt folgende Werte:
Phenol durch HPLC: < 0,1 Gew.-%;
4-Phenolsulfonsäure durch HPLC: 7,8 Gew.-%;
freier Formaldehyd: < 20 ppm;

### 2. Pharmakologische Untersuchungen

### Verfahren zur Bestimmung der antiviralen Aktivität

In der Untersuchung wird bestimmt, ob ein Kondensationsprodukt antivirale Aktivität gegen verschiedene Viren aufweist und welche Menge antiviraler Substanz benötigt wird, um eine 50% Reduktion der Virusreplikation zu bewirken.

Die Virusgebrauchsverdünnung wird an Hand einer Endpunkttitration des angezüchteten Virusisolates ermittelt. Bei dieser Titration wird die Virusmenge ermittelt, bei der 50% der Ansätze aus der Virusverdünnung infiziert bzw. nicht infiziert sind (= infektiöse Dosis 50% = TCID₅₀/ml).

Aus der zu testenden Substanz wird eine um Faktor zwei ansteigende Verdünnungsreihe hergestellt. Danach wird eine definierte Menge Virus zugegeben.

Das Stoff/Virus-Gemisch wird auf Monolayer aus geeigneten Zellen gegeben. Nach einer vom Virus abhängigen Inkubationszeit erfolgt eine Beurteilung der virusbedingten zytopathogenen Veränderung (CPE). Zur Ermittlung der Ergebnisse wird eine Färbung mittels Antikörper gegen das eingesetzte Virus angeschlossen. Dabei erfolgt eine prozentuale Abschätzung des CPE im Vergleich zur Viruskontrolle, die als 100% gesetzt wird. Bei der Färbung wird eine photometrische Auswertung durchgeführt. Mittels linearer Regression unter Verwendung eines Computerprogramms wird die Konzentration, bei der eine 50%ige Reduktion der Virusreplikation von Patientenisolaten bewirkt wird ("IC₅₀"), errechnet.

Die Konzentration an Kondensationsprodukt, die die Zellviabilität um 50% reduziert, wird "TC₅₀" (= toxic concentration) genannt. TC₅₀ zeigt die toxischen Effekte des Reaktionsgemisches an.

Das Verhältnis TC₅₀ / IC₅₀ (therapeutisches Index "TI" genannt) zeigt die spezifische Aktivität eines Kondensationsproduktes gegen ein gegebenes Virus.

### Endpunkttitration des Virus

### Vorbereitung und Verteilung der Zellsuspensionen

### 1. Allgemein

- aus einer Vero-Zellkulturflasche (75cm², ca. 9x10⁶ Zellen) mit konfluentem Zellrasen können ca. 100ml Zellsuspension hergestellt werden.
- pro Mikrotiterplatte werden 5ml Zellsuspension benötigt

### 2. Herstellung der Zellsuspensionen

- die Zellkultur wird trypsiniert, homogenisiert und in Wachstumsmedium überführt

### 3. Verteilung der Zellsuspension

- 50µl Zellsuspension pro Loch ansetzen
- Platten dann bis zur weiteren Benutzung im Brutschrank aufbewahren

### Titration der Viren

- Herstellung einer 1:10 Verdünnungsreihe
- jeweils 50µl Verdünnung pro Loch im 8-fach Ansatz in eine mit Zellsuspension vorbereitete Platte pipettieren
- je nach Virus die Platte im Brutschrank für einige Tage inkubieren
- nach der Inkubationszeit Platten mikroskopisch nach CPE beurteilen

### Testvorbereitung zur Bestimmung der antiviralen Aktivität

- Pro zu untersuchendes Virus muss eine 96-Loch Platte mit entsprechenden Zellen vorbereitet werden
- Beschriftung der Platten.
   ➢ 1. Reihe ZK (Zellkontrolle)
   ➢ 2. Reihe VK (Viruskontrolle)
   ➢ 3. - 10. Reihe Stoffkonzentrationen
   ➢ am Rand Virus und Datum

### Testdurchführung

mit der zu testenden Substanz eine 1:2 Verdünnungsreihe herstellen
- In Reihe 1 der Platte 100µl Medium zu den Zellen geben (keine Stoff- und Virusverdünnung)
- In Reihe 2 50µl Medium pipettieren (keine Stoffverdünnung)
- Substanzverdünnung Reihe 3-12 im 8-fach Ansatz auf die Platte verteilen Das Einpipettieren in die Platten zügig durchführen, da sonst die Zellen austrocknen
   - eine ausreichende Menge Virussuspension für i.d.R. MOI 0,01 herstellen
   - 50µl Virussuspension ab Reihe 2 pipettieren
   - Platte bei 37°C im CO₂-Bruschrank für 2 Tage inkubieren

### Testauswertung

- Nach Ende der Inkubationszeit die Platte mikroskopisch auf CPE auswerten. Dabei entspricht die Viruskontrolle 100%. Für alle Substanzverdünnungen wird, durch Vergleich mit der Viruskontrolle, der Umfang der Zellzerstörung als Prozentzahl angegeben. Nach der visuellen Auswertung kann eine Färbung angeschlossen werden

Färbung mit virusspezifischen Antikörpern
unter der Sterilwerkbank Überstand aus Mikrotiterplatten absaugen
- Fixation der Zellen mit Aceton/Methanol
- Flüssigkeit absaugen
- Verdünnung der virusspezifischen Antikörper mit Blocking Solution. Pro Kavität werden 50µl eingesetzt. Die optimale Konzentration für den Antikörper wird für jede neue Charge mittels Titration ermittelt. Inkubation 1h bei 37°C
- 3x Waschen mit Waschpuffer
- biotinylierte Anti-IgG-Antikörper werden in Waschpuffer verdünnt und je 50µl in jede Kavität einpipettiert. Die optimale Konzentration für den Antikörper wird für jede neue Charge mittels Titration ermittelt.
- Inkubation 1h 37°C
- 3x Waschen
- das Streptavidin/Peroxidase Konjugat wird im Waschpuffer verdünnt und 50µl pro Kavität eingesetzt. Die optimale Konzentration des Konjugates wird für jede neue Charge mittels Titration ermittelt.
- Inkubation für 30Min bei 37°C
- 3x Waschen
- in jede Kavität werden 50µl Substrat-Lösung einpipettiert
- bei Gebrauch eines löslichen Substrates auf einer separaten Platte 2 Reihen mit 50µl Substrat pipettieren (=Substratleerwert)
- Inkubation 10Min bei RT
- Zum Abstoppen bei löslichem Substrat 100ml 1 N Schwefelsäure zugeben
- Auswertung photometrisch bei einer Wellenlänge von 450nm und einer Referenzwellenlänge von 630nm
- die Auswertung sollte innerhalb einer Stunde nach Ende des Tests erfolgen

### Kriterien zur analytischen Freigabe der Ergebnisse:

- die Zellkontrolle muss einen konfluenten, morphologisch unauffälligen Zellrasen zeigen
- die Viruskontrolle wird als 100% CPE gesetzt
- bei der Berechnung des IC₅₀-Wertes darf "r" nicht kleiner als 0,90 sein

### Auswertung und Dokumentation

### visuelle Auswertung

- bei der visuellen Auswertung wird die Ausprägung des CPE bzw. der Färbung im Vergleich mit der Viruskontrolle abgelesen und als %-Wert protokolliert
- Dabei muss die ZK einen konfluenten Zellrasen aufweisen
- grundsätzlich alle Vertiefungen begutachten

### photometrische Auswertung

- bei der photometrischen Auswertung wird aus allen 8-fach Bestimmungen jeweils ein Mittelwert errechnet
- der Mittelwert der Substratleerwerte wird von allen errechneten Mittelwerten subtrahiert
- der OD-Wert der VK entspricht 100%
- mittels Dreisatzrechnung werden die %-Werte der eingesetzten Stoffverdünnungen berechnet

### Berechnung des IC₅₀-Wertes

- aus den 8 Einzelwerten der Kontrollen bzw. der Substanzbestimmungen den jeweiligen Mittelwert ermitteln
- den Substratleerwert von allen Werten subtrahieren
- bei der antiviralen Bestimmung entspricht der Wert der VK 100%
- für die einzelnen Werte der Substanzbestimmungen den %-Wert in Bezug auf die jeweilige Kontrolle berechnen
- ermittelte %-Werte in das Computerprogramm Calcusyn for Windows (Fa. Biosoft) einsetzen und den IC₅₀-Wert berechnen.

### Qualitätskontrolle

In jedem Ansatz wird eine Substanz mit bekannter antiviraler Aktivität gegenüber dem Prüfvirus mitgeführt. In jedem Ansatz wird eine Zell- und Viruskontrolle mitgeführt.

### Beispiele antiviraler Aktivität von Kondensationsprodukten

### Kondensationsprodukt aus Beispiel 1

| **Virus** | **IC₅₀ (µg/ml)** | **TC₅₀ (µg/ml)** | **TI** |
|---|---|---|---|
| SARS-assoziiertes Coronavirus (SARS-CoV) | 67 | 1633 | 24 |
| Herpes simplex Virus 1 (HSV-1) | 3,1 | 1848 | 596 |
| Humanes Zytomegalievirus (HCMV) | 0,73 | 1848 | 2532 |
| Respiratorisches Syncytialvirus (RSV) | 38 | 1633 | 43 |
| Humanes Immundefizienzvirus Typ 1 (HIV-1) | 1,1 | 1359 | 1235 |

### Kondensationsprodukt aus Vergleichsbeispiel 2

| **Virus** | **IC₅₀ (µg/ml)** | **TC₅₀ (µg/ml)** | **TI** |
|---|---|---|---|
| SARS-assoziiertes Coronavirus (SARS-CoV) | 189 | 476 | 2,5 |
| Herpes simplex Virus 1 (HSV-1) | 10,41 | 99,2 | 9,5 |
| Humanes Zytomegalievirus (HCMV) | 1,59 | 99,2 | 62,39 |
| Respiratorisches Syncytialvirus (RSV) | 114 | 476 | 4,2 |
| Humanes Immundefizienzvirus Typ 1 (HIV-1) | 10,8 | 405 | 37,7 |

Der Vergleich der Beispiele 1 und 2 zeigt, dass die erfindungsgemäßen Kondensationsprodukte für eine Vielzahl von Viren einen deutlich verringerten IC₅₀-Wert und einen deutlich höheren TC₅₀-Wert und somit auch einen deutlich verbesserten TI-index aufweisen als die aus dem Stand der Technik bekannten Kondensationsprodukte. Die erfindungsgemäßen Kondensationsprodukte zeigen somit eine verbesserte pharmazeutische Wirksamkeit.

## Patentansprüche

1. Kondensationsprodukt erhältlich durch Umsetzung von
a1) mindestens einem Aromaten oder Heteroaromaten, wobei der Aromat oder Heteroaromat mit mindestens einer Hydroxy- (-OH) und mindestens einer Carboxy-Gruppe (-COOH) substituiert ist, und wobei die Hydroxy- und/oder Carboxy-Gruppe in Salzform vorliegen können,
a2) mindestens einer Carbonylverbindung, ausgewählt aus Aldehyden und Ketonen,
a3) mindestens einem Sulfonierungsmittel,
a4) mindestens einem Harnstoffderivat, und
a5) mindestens einem weiteren Aromaten oder Heteroaromaten, ausgewählt aus Benzol, Naphthalin, Anthracen, aromatischen Alkoholen, aromatischen Ethern und aromatischen Sulfonen, wobei die vorgenannten Verbindungen substituiert sein können und diese Verbindungen nicht mit mindestens einer Hydroxy- (-OH) und mindestens einer Carboxy-Gruppe (-COOH), oder die Salzform dieser Verbindungen, substituiert sind,
oder ein physiologisch verträgliches Salz davon,
wobei die Umsetzung der einzelnen Komponenten a1) und a2) sowie a3) bis a5) in einer oder in mehreren Stufen erfolgen kann, wobei
zunächst mindestens eine Komponente a1) mit mindestens einer Komponente a3) umgesetzt werden und danach im selben Gefäß ohne vorherige Isolierung mit mindestens einer Komponente a2), mindestens einer Komponente a4) und mindestens einer Komponente a5) umgesetzt werden oder
die Reihenfolge der Zugabe der Komponenten a1) und a5) vertauscht ist oder
die Komponenten a1) und a5) mindestens einmal in Form eines Gemisches zugegeben werden oder
mindestens eine Komponente a1) mit mindestens einer Komponente a3) umgesetzt werden, das Produkt isoliert und dann mit dem Umsetzungsprodukt von mindestens einer Komponente a2), mindestens einer Komponente a4) und mindestens einer Komponente a5) umgesetzt wird,
unter der Voraussetzung, dass die Komponente a1) nicht Salicylsäure oder Sulfosalicylsäure ist, wenn die Komponente a4) Melamin ist,
zur Verwendung als Arzneimittel.

2. Kondensationsprodukt gemäß Anspruch 1, **dadurch gekennzeichnet, dass** in der Komponente a1) der Aromat Benzol ist.

3. Kondensationsprodukt gemäß Anspruch 1 oder 2, **dadurch gekennzeichnet, dass** die Komponente a1) Gallussäure und/oder Salicylsäure oder ein Salz davon ist.

4. Kondensationsprodukt gemäß einem der Ansprüche 1 bis 3, erhältlich durch Umsetzung von
a1) Gallussäure oder einem Salz der Gallussäure,
a2) mindestens einem Aldehyd ausgewählt aus Formaldehyd, Acetaldehyd und Propionaldehyd,
a3) konzentrierter Schwefelsäure,
a4) mindestens einem Harnstoffderivat ausgewählt aus Harnstoff, Melamin, 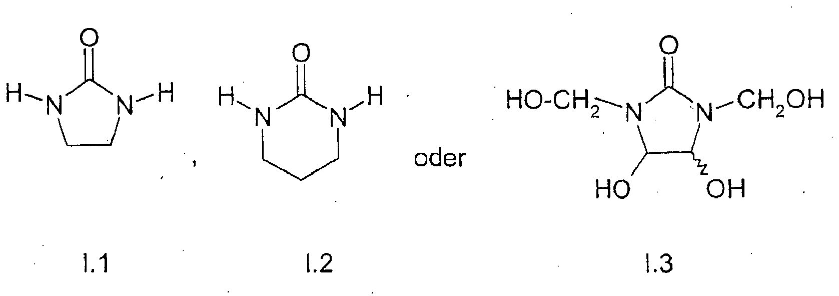
a5) Phenol.

5. Kondensationsprodukt gemäß einem der Ansprüche 1 bis 4, **dadurch gekennzeichnet, dass** das Kondensationsprodukt als Gemisch mit mindestens einem Gerbstoff, insbesondere einem synthetischen oder pflanzlichen Gerbstoff vorliegt.

6. Verwendung eines Kondensationsproduktes nach einem der Ansprüche 1 bis 5 zur Herstellung von einem Medikament zur Prophylaxe und/oder Behandlung von Genitalwarzen, Gebärmutterhalskrebs, allergischen oder nicht-allergischen Ekzemen, insbesondere Neurodermitis (endogenes Ekzem), Wundsein, Juckreiz, entzündlichen Erkrankungen, Autoimmunerkrankungen, insbesondere Arthritis, Rheuma, melanomen Karzinomen, Entzündungen der Haut, Herpes, insbesondere Herpes labilis oder Herpes simplex, Windpocken oder Aids.

7. Verwendung eines Kondensationsproduktes nach einem der Ansprüche 1 bis 5 zur Herstellung von einem Medikament, das ein antivirales Agens ist, vorzugsweise gegenüber humanen Papillomviren, speziell Typ 16, 18, 6 und 11, endogenen Retroviren, insbesondere HERV-Typ, Herpes-Viren, HCMV-Viren, HIV-Viren, Corona-Viren, Flaviviren, Togaviren oder Paramoxyviren.

8. Pharmazeutische Zusammensetzung umfassend eine wirksame Menge von mindestens einem Kondensationsprodukt gemäß einem der Ansprüche 1 bis 5 und einem physiologisch verträglichen Träger.

9. Pharmazeutische Zusammensetzung gemäß Anspruch 8, wobei die pharmazeutische Zusammensetzung in Form einer Pille, Tablette, Lutschtablette, Granulat, Kapsel, harte oder weiche Gelatinekapsel, wässrigen Lösung, alkoholischen Lösung, ölartigen Lösung, Sirup, Emulsion, Suspension, Zäpfchen, Pastille, Lösung zur Injektion oder Infusion, Salbe, Tinktur, Creme, Lotion, Puder, Spray, eines transdermalen therapeutischen Systems, Nasenspray, Aerosol, Aerosol-Mischung, Mikrokapsel, Implantat, Stab, Pflaster oder Gel vorliegt oder dass die pharmazeutische Zusammensetzung Bestandteil von Health Care-Produkten wie Sonnenschutzcremes, Nasensprays, Mundwässer, Zahnpasten, Pflastern, (Feucht)-Tüchern, Waschlotionen oder Shampoos ist.

10. Verwendung eines Kondensationsproduktes gemäß einem der Ansprüche 1 bis 5 zur Desinfektion, als Desinfektionsmittel oder Bestandteil eines Desinfektionsmittels.

11. Verwendung gemäß Anspruch 10 im Bereich von Krankenhäusern, insbesondere Krankenhaus-Intensivstationen, Toiletten, Waschräumen, Haushalten, der Lebensmittelproduktion oder in Stallungen oder Käfigen von Tieren, insbesondere von Vögeln, Schweinen oder Rindern.

12. Desinfektionsmittel enthaltend mindestens ein Kondensationsprodukt gemäß einem der Ansprüche 1 bis 5.

13. Verfahren zur Herstellung eines Kondensationsproduktes gemäß einem der Ansprüche 1 bis 5, **dadurch gekennzeichnet, dass** die einzelnen Komponenten a1) bis a5) in einer oder in mehreren Stufen miteinander umgesetzt werden, wobei zunächst mindestens eine Komponente a1) mit mindestens einer Komponente a3) umgesetzt werden und danach im selben Gefäß ohne vorherige Isolierung mit mindestens einer Komponente a2), mindestens einer Komponente a4) und mindestens einer Komponente a5) umgesetzt werden oder
die Reihenfolge der Zugabe der Komponenten a1) und a5) vertauscht ist oder
die Komponenten a1) und a5) mindestens einmal in Form eines Gemisches zugegeben werden oder
mindestens eine Komponente a1) mit mindestens einer Komponente a3) umgesetzt werden, das Produkt isoliert und dann mit dem Umsetzungsprodukt von mindestens einer Komponente a2), mindestens einer Komponente a4) und mindestens einer Komponente a5) umgesetzt wird.

## Claims

1. A condensation product obtainable by reaction of
a1) at least one aromatic or heteroaromatic, where the aromatic or heteroaromatic is substituted by at least one hydroxyl group (-OH) and at least one carboxyl group (-COOH), and where the hydroxyl and/or carboxyl group can be present in salt form,
a2) at least one carbonyl compound, selected from aldehydes and ketones,
a3) at least one sulfonating agent,
a4) at least one urea derivative, and
a5) at least one further aromatic or heteroaromatic, selected from benzene, naphthalene, anthracene, aromatic alcohols, aromatic ethers and aromatic sulfones, where the abovementioned compounds can be substituted and these compounds are not substituted by at least one hydroxyl group (-OH) and at least one carboxyl group (-COOH), or the salt form of these compounds,
or a physiologically tolerable salt thereof,
wherein the individual components a1) and a2) and also a3) to a5) can be reacted in one or in a number of stages, where
initially at least one component a1) is reacted with at least one component a3) and then, in the same vessel without prior isolation, with at least one component a2), at least one component a4) and at least one component a5) or
the order of the addition of components a1) and a5) is changed or
the components a1) and a5) are at least once added in the form of a mixture or
at least one component a1) is reacted with at least one component a3), the product is isolated and then reacted with the reaction product of at least one component a2), at least one component a4) and at least one component a5),
with the precondition that the component a1) is not salicylic acid or sulfosalicylic acid if the component a4) is melamine,
for use as a drug.

2. The condensation product according to claim 1, wherein the aromatic in component a1) is benzene.

3. The condensation product according to claim 1 or 2, wherein component a1) is gallic acid and/or salicylic acid or a salt thereof.

4. The condensation product according to one of claims 1 to 3, obtainable by reaction of
a1) gallic acid or a salt of gallic acid,
a2) at least one aldehyde selected from formaldehyde, acetaldehyde and propionaldehyde,
a3) concentrated sulfuric acid,
a4) at least one urea derivative selected from urea, melamine, 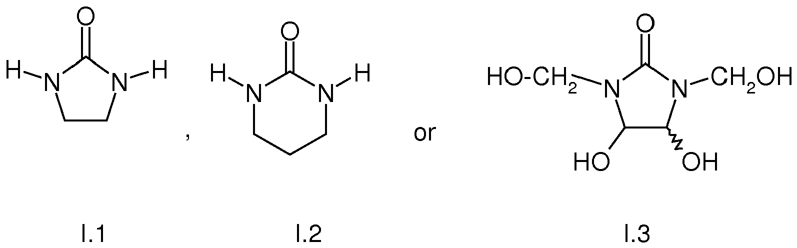
a5) phenol.

5. The condensation product according to one of claims 1 to 4, wherein the condensation product is present as a mixture with at least one tanning agent, in particular a synthetic or plant tanning agent.

6. The use of a condensation product according to one of claims 1 to 5 for the production of a medicament for the prophylaxis and/or treatment of genital warts, cancer of the uterine cervix, allergic or nonallergic eczema, in particular neurodermatitis (endogenous eczema), diaper rash, pruritus, inflammatory diseases, autoimmune diseases, in particular arthritis, rheumatism, melanomatous carcinomas, inflammations of the skin, herpes, in particular herpes labilis or herpes simplex, chickenpox or Aids.

7. The use of a condensation product according to one of claims 1 to 5 for the production of a medicament which is an antiviral agent, preferably against human papillomaviruses, especially type 16, 18, 6 and 11, endogenous retroviruses, in particular HERV type, herpes viruses, HCMV viruses, HIV viruses, coronaviruses, flaviviruses, togaviruses or paramyxoviruses.

8. A pharmaceutical composition comprising an efficacious amount of at least one condensation product according to one of claims 1 to 5 and a physiologically tolerable vehicle.

9. The pharmaceutical composition according to claim 8, where the pharmaceutical composition is present in the form of a pill, tablet, lozenge, granules, capsule, hard or soft gelatin capsule, aqueous solution, alcoholic solution, oily solution, syrup, emulsion, suspension, suppository, pastille, solution for injection or infusion, ointment, tincture, cream, lotion, cosmetic powder, spray, of a transdermal therapeutic system, nasal spray, aerosol, aerosol mixture, microcapsule, implant, rod, patch or gel or wherein the pharmaceutical composition is a constituent of healthcare products such as sunscreen creams, nasal sprays, mouthwashes, toothpastes, plasters, (wet) wipes, cleansing lotions or shampoos.

10. The use of a condensation product according to one of claims 1 to 5 for disinfection, as a disinfectant or constituent of a disinfectant.

11. The use according to claim 10 in the hospital field, in particular hospital intensive care units, toilets, washrooms, households, food production or in stables or cages of animals, in particular of birds, pigs or cattle.

12. A disinfectant comprising at least one condensation product according to one of claims 1 to 5.

13. A process for the preparation of a condensation product according to one of claims 1 to 5, wherein the individual components a1) to a5) are reacted with one another in one or in a number of stages, where
initially at least one component a1) is reacted with at least one component a3) and then, in the same vessel without prior isolation, with at least one component a2), at least one component a4) and at least one component a5) or
the order of the addition of components a1) and a5) is changed or
the components a1) and a5) are at least once added in the form of a mixture or
at least one component a1) is reacted with at least one component a3), the product is isolated and then reacted with the reaction product of at least one component a2), at least one component a4) and at least one component a5).

## Revendications

1. Produit de condensation pouvant être obtenu par mise en réaction de
a1) au moins un composé aromatique ou composé hétéro-aromatique, le composé aromatique ou le composé hétéroaromatique étant substitué par au moins un groupe hydroxy (-OH) et au moins un groupe carboxy (-COOH), et le groupe hydroxy et/ou le groupe carboxy pouvant se trouver sous forme de sel,
a2) au moins un composé carbonyle, choisi parmi des aldéhydes et des cétones,
a3) au moins un agent de sulfonation,
a4) au moins un dérivé d'urée, et
a5) au moins un autre composé aromatique ou composé hétéroaromatique, choisi parmi le benzène, le naphtalène, l'anthracène, des alcools aromatiques, des éthers aromatiques et des sulfones aromatiques, les composés précités pouvant être substitués et ces composés n'étant pas substitués par au moins un groupe hydroxy (-OH) et au moins un groupe carboxy (-COOH), ou la forme sel de ces composés,
ou sel physiologiquement acceptable de celui-ci,
la réaction des composants individuels a1) et a2) ainsi que a3) à a5) pouvant s'effectuer en une ou en plusieurs étapes,
en faisant réagir d'abord au moins un composant a1) avec au moins un composant a3) et ensuite dans le même récipient, sans isolement préalable, avec au moins un composant a2), au moins un composant a4) et au moins un composant a5) ou
en changeant l'ordre de l'addition des composants a1) et a5) ou
en ajoutant les composants a1) et a5) au moins une fois sous forme d'un mélange ou
en faisant réagir au moins un composant a1) avec au moins un composant a3), puis en isolant le produit et ensuite en le faisant réagir avec le produit de réaction d'au moins un composant a2), au moins un composant a4) et au moins un composant a5),
étant entendu que le composant a1) n'est pas l'acide salicylique ou l'acide sulfosalicylique lorsque le composant a4) est la mélamine,
pour utilisation en tant que médicament.

2. Produit de condensation selon la revendication 1, **caractérisé en ce que** dans le composant a1) le composé aromatique est le benzène.

3. Produit de condensation selon la revendication 1 ou 2, **caractérisé en ce que** le composant a1) est l'acide gallique et/ou l'acide salicylique ou un sel d'un tel acide.

4. Produit de condensation selon l'une quelconque des revendications 1 à 3, pouvant être obtenu par mise en réaction
a1) d'acide gallique ou d'un sel de l'acide gallique,
a2) d'au moins un aldéhyde choisi parmi le formaldéhyde, l'acétaldéhyde et le propionaldéhyde,
a3) d'acide sulfurique concentré,
a4) d'au moins un dérivé d'urée, choisi parmi l'urée, la mélamine, 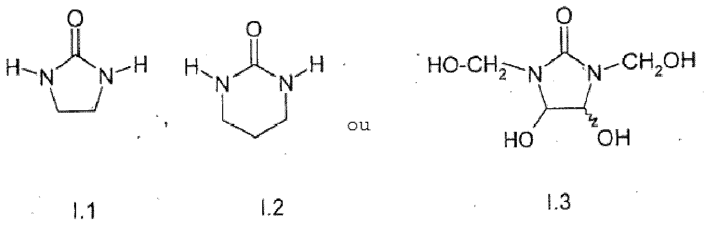
a5) de phénol.

5. Produit de condensation selon l'une quelconque des revendications 1 à 4, **caractérisé en ce que** le produit de condensation se trouve sous forme de mélange avec au moins un tanin, en particulier un tanin végétal ou synthétique.

6. Utilisation d'un produit de condensation selon l'une quelconque des revendications 1 à 5, pour la fabrication d'un médicament destiné à la prophylaxie et/ou au traitement de verrues génitales, de cancer du col utérin, d'eczémas allergiques ou non allergiques, notamment de névrodermite (eczéma endogène), d'intertrigo, de prurit, de maladies inflammatoires, de maladies auto-immunes, en particulier de l'arthrite, de rhumatisme, de carcinomes mélanomiques, d'inflammations de la peau, d'herpès, notamment de l'herpès de la lèvre ou herpes simplex, de la varicelle ou du sida.

7. Utilisation d'un produit de condensation selon l'une quelconque des revendications 1 à 5, pour la fabrication d'un médicament qui est un agent antiviral, de préférence contre des papillomavirus humains, en particulier les types 16, 18, 6 et 11, des rétrovirus endogènes, en particulier le type HERV, des herpesvirus, virus HCMV, virus VIH, coronavirus, flavivirus, togavirus ou paramoxyvirus.

8. Composition pharmaceutique comprenant une quantité efficace d'au moins un produit de condensation selon l'une quelconque des revendications 1 à 5 et un véhicule physiologiquement acceptable.

9. Composition pharmaceutique selon la revendication 8, la composition pharmaceutique étant sous forme d'une pilule, d'un comprimé, d'un comprimé à sucer, d'un granulé, d'une capsule, d'une gélule ou d'une capsule de gélatine molle, d'une solution aqueuse, d'une solution alcoolique, d'une solution huileuse, d'un sirop, d'une émulsion, d'une suspension, d'un suppositoire, d'une pastille, d'une solution pour injection ou perfusion, d'une pommade, d'une teinture, d'une crème, d'une lotion, d'une poudre, d'une composition à pulvériser en aérosol, d'un système thérapeutique transdermique, d'une composition pour pulvérisation nasale, d'un aérosol, d'un mélange pour aérosol, d'une microcapsule, d'un implant, d'une tige, d'un pansement ou d'un gel ou la composition pharmaceutique faisant partie de produits de soin de santé tels que des crèmes de protection solaire, produits pour pulvérisation nasale, bains de bouche, pâtes dentifrice, pansements, lingettes (humides), lotions lavantes ou shampooings.

10. Utilisation d'un produit de condensation selon l'une quelconque des revendications 1 à 5, pour la désinfection, en tant que désinfectant ou composant d'un désinfectant.

11. Utilisation selon la revendication 10, dans le secteur des hôpitaux, en particulier des unités hospitalières de soins intensifs, des toilettes, des salles d'eau, de la maison, de la fabrication des produits alimentaires ou dans des bâtiments ou cages hébergeant des animaux, en particulier des oiseaux, porcins ou bovins.

12. Désinfectant contenant au moins un produit de condensation selon l'une quelconque des revendications 1 à 5.

13. Procédé pour la préparation d'un produit de condensation selon l'une quelconque des revendications 1 à 5, **caractérisé en ce qu'**on fait réagir entre eux les composants individuels a1) à a5) en une ou en plusieurs étapes,
en faisant réagir d'abord au moins un composant a1) avec au moins un composant a3) et ensuite dans le même récipient, sans isolement préalable, avec au moins un composant a2), au moins un composant a4) et au moins un composant a5) ou
en changeant l'ordre de l'addition des composants a1) et a5) ou
en ajoutant les composants a1) et a5) au moins une fois sous forme d'un mélange ou
en faisant réagir au moins un composant a1) avec au moins un composant a3), puis en isolant le produit et ensuite en le faisant réagir avec le produit de réaction d'au moins un composant a2), au moins un composant a4) et au moins un composant a5).
